(19) Europäisches Patentamt
European Patent Office
Office européen des brevets

(11) **EP 1 880 718 B1**

(12) **EUROPÄISCHE PATENTSCHRIFT**

(45) Veröffentlichungstag und Bekanntmachung des Hinweises auf die Patenterteilung:
**21.09.2011 Patentblatt 2011/38**

(51) Int Cl.:
***A61K 9/50*** *(2006.01)*

(21) Anmeldenummer: **06014244.5**

(22) Anmeldetag: **10.07.2006**

(54) **Pharmazeutische Zubereitung für die orale Verabreichung mit gesteuerter Wirkstofffreisetzung im Dünndarm und Verfahren zu ihrer Herstellung**

Pharmaceutical preparation for oral administration with controlled drug release in the small intestine colon and process of prepartion thereof

Préparation pharmaceutique pour administration orale avec libération controlée d'actif dans l'intestin grêle et procédé de préparation

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK RS**

(43) Veröffentlichungstag der Anmeldung:
**23.01.2008 Patentblatt 2008/04**

(73) Patentinhaber: **Dr. R. Pfleger Chemische Fabrik GmbH**
**96052 Bamberg (DE)**

(72) Erfinder:
• **Jung, Gerd**
**96149 Breitengüssbach (DE)**

• **Schaupp, Albert**
**96129 Strullendorf (DE)**

(74) Vertreter: **TER MEER - STEINMEISTER & PARTNER GbR**
**Patentanwälte**
**Mauerkircherstrasse 45**
**81679 München (DE)**

(56) Entgegenhaltungen:
**EP-A2- 0 335 560    EP-A2- 1 086 694**
**WO-A-01/68058       WO-A-98/51287**
**WO-A1-01/87269**

Bemerkungen:
Die Akte enthält technische Angaben, die nach dem Eingang der Anmeldung eingereicht wurden und die nicht in dieser Patentschrift enthalten sind.

EP 1 880 718 B1

## Beschreibung

Die vorliegende Erfindung betrifft eine pharmazeutische Zubereitung für die orale Verabreichung mit gesteuerter Wirkstofffreisetzung im Dünndarm, auf der Grundlage von mit mindestens einem Wirkstoff versehenen Wirkstoffträgern, die mit einer inneren Schicht zur Steuerung der Wirkstofffreisetzung und einer darauf angeordneten magensaftresistenten Überzugsschicht versehen sind, sowie ein Verfahren zu ihrer Herstellung.

Pharmazeutische Zubereitungen für die orale Verabreichung mit gesteuerter Wirkstofffreisetzung im Dünndarm sind bereits bekannt.

So beschreibt die DE 198 23 940 A1 enterische Fluoxetinpellets, welche einen neutralen Kern umfassen, auf den eine Fluoxetinschicht aufgebracht worden ist, der sich eine Trennschicht anschließt, auf welche eine enterische Schicht aufgebracht ist, die gegebenenfalls mit eine Deckschicht versehen ist. Dabei dient die enterische Schicht dazu, sicherzustellen, daß der Wirkstoff den Magen des Patienten unverändert passiert und sich erst dann auflöst, wenn der Wirkstoff den Magen verläßt und in den Dünndarm eintritt.

Gegenstand der EP 0 941 070 B1 ist eine Lutschtablette zur modifizierten Freisetzung von Wirkstoffen im Gastrointestinaltrakt, welche eine Wirkstoffzubereitung in Form von mindestens zweischichtig umhüllten Partikeln enthält, wobei eine äußere Umhüllungsschicht speichelresistent, jedoch magensaftlöslich, und eine innere Umhüllungsschicht in wässrigen Medien weitgehend zerfallsresistent ist, eine retardierende Wirkstofffreisetzung durch Diffusion jedoch zuläßt. Gemäß einer weiteren Ausführungsform umfassen die zweischichtig umhüllten Partikel eine äußere Umhüllungsschicht, die speichelresistent, jedoch magensaftlöslich ist, und eine innere Umhüllungsschicht, die magensaftresistent, jedoch dünndarmlöslich ist.

Aus der US-Patentanmeldung US 2004/0142035 A1 ist eine pharmazeutische Zubereitung bekannt, welche einen Kern, der ein oder mehrere pharmazeutische Wirkstoffe enthält, und eine den Kern umhüllende Überzugsschicht aufweist, wobei die Überzugsschicht eine Kombination aus zwei oder mehr enterischen Beschichtungsmaterialien umfaßt, von denen mindestens zwei der enterischen Beschichtungsmaterialien sich bei unterschiedlichen pH-Werten lösen, um in dieser Weise ein in Abhängigkeit von dem pH-Wert gesteuertes Freisetzungsprofil des Wirkstoffes zu erreichen. Dieser Aufbau dient dazu, zu verhindern, daß ein signifikanter Teil des Wirkstoffes nach dem Durchlaufen des Dünndarms noch in dem Präparat vorhanden ist und verlorengeht.

Die herkömmlichen pharmazeutischen Zubereitungen für die orale Verabreichung mit gesteuerter Wirkstofffreisetzung im Dünndarm haben sich aber nicht als vollständig zufriedenstellend erwiesen, weil sie bei der Wirkstofffreisetzung im Dünndarm zunächst einen starken Anstieg der Wirkstofffreisetzung mit entsprechend hohem Blutspiegel ergeben, was in vielen Fällen zu einer Verstärkung der Nebenwirkungsrate und -intensität führt. Bei im weiteren genannten Substanzen betrifft dies z.B. insbesondere Mundtrockenheit, Akkomodationsstörungen, Magen-Darm-Probleme oder zentralnervöse Effekte. Dies ist besonders nachteilig dann, wenn die gesamte Dosis des Wirkstoffes nur einmal täglich appliziert werden soll. Weiterhin wird beobachtet, daß es nach oraler Gabe z.B. von Anticholinergika zu einer starken interindividuellen Streuung der Plasmaspiegelverläufe kommt.

Andererseits ergeben sich durch die Verwendung von enterischen Materialien mit unterschiedlichen Löslichkeiten bei unterschiedlichen pH-Werten, wie es die Lehre der US 2004/0142035 A1 vorsieht, Einschränkungen bei der Materialwahl einerseits und andererseits keine gleichmäßige Wirkstofffreisetzung bei pH-Schwankungen.

WO01088058 offenbart eine mehrschichtigen Arzneiform, die aufgebaut ist aus a) einem Kern mit einem pharmazeutischen Wirkstoff b) einem inneren Überzug aus einem Copolymeren oder einer Mischung von Copolymeren, die sich aus 85 bis 98 Gew.-% radikalisch polymerisierten C1- bis C4-Aikylestern der Acryl-oder der Methacrylsäure und 15 bis 2 Gew.-% (Meth) acrylat-Monomeren mit einer quaternären Ammoniumgruppe im Alkylrest zusammensetzen und einem c) einem äusseren Überzug aus einem Copolymeren, das sich aus 75 bis 95 Gew.-% radikalisch polymerisierten CI-bis C4-Alkylestern der Acryl-oder der Methacrylsäure und 5 bis 25 Gew.-% (Meth) acrylat-Monomeren mit einer anionischen Gruppe im Alkylrest zusammensetzt zur Herstellung einer Arzneiform.

EP-A-1086694 offenbart ein Pellet umfassend a) einen inerten Kern, b) eine über dem inerten Kern angeordnete Schicht enthaltend eine Säurelabile Benzimidazolverbindung c) eine oder mehrere Zwischenschichten die i) eine inerte Beschichtung, das ein wasserlösliches Polymer umfasst und ii) ein modifiziertes Freisetzungssystem, das ein wasserlösliches Polymer umfasst und d) eine Aussenschicht.

Die Aufgabe der vorliegenden Erfindung besteht also darin, eine pharmazeutische Zubereitung der eingangs angegebenen Art für die orale Verabreichung von Wirkstoffen mit gesteuerter Wirkstofffreisetzung im Dünndarm bereitzustellen, welche im Gegensatz zu herkömmlichen Formulierungen einen geringeren anfänglichen Anstieg der Wirkstofffreisetzung und damit einen niedrigeren anfänglichen Plasmapeak des Wirkstoffes aufweist, jedoch im weiteren Verlauf eine niedrigere aber dafür gleichförmige Plasmakonzentration des Wirkstoffes sicherstellt und damit zu einer geringeren Nebenwirkungsrate und Nebenwirkungsintensität und einer geringeren interindividuellen Streuung der Plasmaspiegelverläufe führt, insbesondere dann, wenn die gesamte Dosis des Wirkstoffes nur einmal täglich appliziert werden soll.

Bei der vorgesehenen Zubereitung wird dies erreicht, wenn bei der in vitro Prüfung der Freisetzung gemäß USP Pharmacopöe die Freisetzungsrate des Wirkstoffes ≥75 % in 60 Minuten beträgt. Insofern ist es ohne Probleme möglich, die erforderliche Dosis für den ganzen Tag mit einer einzigen Verabreichung sicherzustellen.

Es hat sich überraschenderweise gezeigt, daß diese Aufgabe dadurch gelöst werden kann, daß man die mit einem Wirkstoff versehenen Wirkstoffträger mit einer inneren Schicht zur Steuerung der Wirkstofffreisetzung versieht, welche aus mindestens zwei Diffusionsschichten ausgebildet ist, deren Durchlässigkeit für den diffundierenden Wirkstoff von innen nach außen abnimmt und die weiterhin mit einer darauf angeordneten magensaftresistenten Überzugsschicht versehen sind.

Gegenstand der Erfindung ist daher die pharmazeutische Zubereitung gemäß Hauptanspruch und deren Verwendung.

Die Erfindung betrifft somit eine pharmazeutische Zubereitung für die orale Verabreichung mit gesteuerter Wirkstofffreisetzung im Dünndarm, auf der Grundlage von mit mindestens einem Wirkstoff versehenen Wirkstoffträgern, die mit einer inneren Schicht zur Steuerung der Wirkstofffreisetzung und einer darauf angeordneten magensaftresistenten Überzugsschicht versehen sind, die dadurch gekennzeichnet ist, daß die innere Schicht aus mindestens zwei Diffusionsschichten ausgebildet ist, deren Durchlässigkeit für den diffundierenden Wirkstoff von innen nach außen abnimmt.

Bei dieser Ausbildung der mindestens zwei Diffusionsschichten umfassenden inneren Schicht wird es möglich die Wirkstofffreisetzung im Dünndarm gezielt zu bewirken, so daß im Vergleich zu herkömmlichen Produkten dieser Art eine geringere Anfangsgeschwindigkeit der Wirkstofffreisetzung erreicht werden kann, was bei der Applizierung der gesamten Tagesdosis des Wirkstoffes in einer Einmalgabe bei entsprechend niedrigeren, jedoch ausreichenden Blutspiegeln zu einer geringeren interindividuellen Streuung der Plasmaspiegelverläufe und somit zu einer verringerten Nebenwirkungsrate und -intensität führt.

Gemäß der Erfindung sind die mindestens zwei Diffusionsschichten der inneren Schicht zur Steuerung der Wirkstofffreisetzung aus einem im Dünndarmsaft unlöslichen Matrixmaterial gebildet, in das die Durchlässigkeit für den diffundierenden Wirkstoff steuernde, im Dünndarmsaft lösliche und/oder quellbare und/oder wasseraufnehmende Porenbildner eingelagert sind. Dabei können die Diffusionsschichten gleiche oder verschiedenartige Matrixmaterialien und/oder einen oder mehrere, gleichartige oder verschiedenartige Porenbildner enthalten.

Erfindungsgemäß wird also die Durchlässigkeit der Diffusionsschichten für den diffundierenden Wirkstoff vorzugsweise über die Art, die Menge und/oder die Teilchengröße und/oder die Löslichkeit und/oder die Quellbarkeit und/oder das Wasseraufnahmevermögen der in dem Matrixmaterial vorliegenden Porenbildner gesteuert.

Erfindungsgemäß weist die innere Schicht zur Steuerung der Wirkstofffreisetzung eine innere und eine äußere Diffusionsschicht auf, dabei ist bei identischem Porenbildnermaterial und Matrixmaterial die Teilchengröße und/oder die Konzentration der Porenbildner in der inneren Diffusionsschicht größer als in der äußeren Diffusionsschicht. Gemäß einer bevorzugten Ausführungsform liegt bei im Dünndarmsaft löslichen Porenbildnern das Verhältnis der Porenbildner-Konzentration in der inneren Diffusionsschicht zu der Porenbildner-Konzentration in der äußeren Diffusionsschicht im Bereich von 20:1 bis > 1:1, vorzugsweise im Bereich von 10:1 1 bis 1,1:1.

Das Matrixmaterial der Diffusionsschichten wird vorzugsweise aus der Ethylcellulose, Celluloseacetatphthalat, Celluloseacetatsuccinat, Schellack, Hydroxypropylmethylcelluloseacetatsuccinat, Celluloseacetat, Celluloseacetatpropionat, Hydroxypropylmethylcellulosephthalat, Polyvinylacetat, Polyvinylacetatphthalat, Celluloseacetatbutyrat, Butylmethacrylat-(2-Dimethylaminoethyl)-methacrylat-Methylmethacrylat-Copolymere, Ethylacrylat-Methylmethacrylat-Copolymere, Methacrylsäure-Methylmethacrylat-Copolymere, Methacrylsäure-Ethylacrylat-Copolymere, Ethylacrylat-Methylmethacrylat-Trimethylammoniumethylmethacrylatchlorid-Copolymere, Chitosan, Chitosanacetat, Siliconelastomer-Latexsuspensionen hydriertes Rizinusöl, Stearinsäure, Glycerinmonostearat, Glycerindistearat, Glycerindibehenat, Stearylalkohol, weißes Wachs, gelbes Wachs, hydriertes Pflanzenöl und mikrokristallines Wachs umfassenden Gruppe ausgewählt.

Als Material für die Porenbildner der Diffusionsschichten wählt man Materialien aus der Polyvinylalkohol-Polyethylenglykol-Copolymere, Polyvinylpyrrolidon, Vinylpyrrolidon-Vinylacetat-Copolymere, vernetztes Polyvinylpyrrolidon, mikrokristalline Cellulose, Hydroxypropylcellulose, Natriumcarboxymethylcellulose, Methylcellulose, Hydroxyethylcellulose, Methylethylcellulose, Methylhydroxypropylcellulose, Hydroxypropylmethylcellulose, Hydroxyethylmethylpropylcellulose, Polyethylenglykol, Cellulosepulver, Saccharose, Lactose, Mannitol, Sorbitol und Polysorbat bestehenden Gruppe aus.

Erforderlichenfalls kann das Matrixmaterial der Diffusionsschichten zusätzlich Weichmacher und Trennmittel enthalten, um die Eigenschaft des Matrixmaterials der Diffusionsschichten in der gewünschten Weise einzustellen. Vorzugsweise sieht das Matrixmaterial der Diffusionsschichten als Weichmacher Polyethylenglykol, Propylenglykol, Triethylcitrat, Triacetin, Acetyltributylcitrat, Polysorbate, 2-Pyrrolidon, Sebacinsäuredibutylester, Stearinsäure, Rizinusöl und/oder mittelkettige Triglyceride, und als Trennmittel Talkum, Stearinsäure und deren Salze, Fettalkohole, Mono-, Di- oder Triglyceride mit gerad- und/oder verzweigtkettigen Fettsäuren, hochdisperses Siliciumdioxid, gefälltes Siliciumdioxid, Aluminiumoxid, Kaolin, Maisstärke, Weizenstärke, Reisstärke, Kartoffelstärke, Titandioxid, Siliconemulsionen und/oder Veegum (eine Magnesium-Aluminium-silikat-Dispersion) vor.

Die in der erfindungsgemäßen pharmazeutischen Zubereitung enthaltenen Wirkstoffträger enthalten als Wirk-

stoff Arzneimittehwirkstoffe, deren Wirkung über den Dünndarm sichergestellt werden kann, 4-Diethylamino-2-butinyl-α-phenylcyclohexaneglyconat-Hydrochlorid, Ethyldimethyl-(1-methyl-3,3-diphenylpropyl)-ammoniumcarrageenat, (+)-(R-2-[α-2-(Diisopropylamino)-ethyl]-benzyl]-p-cresol-tartrat, 8-(Cyclopropylmethyl)-6β,7β-epoxy-3α-hydroxy-1αH, 5α)-(S)-tropat-bromid, (1R,3r,5S)-3-[(Hydroxydiphenylacetyl)-oxy]-spiro[8-azoniabicyclo[3.2.1]octan-8,1'-pyrrolidinium]-chlorid, 2-Diethylaminoethyl-(bicyclohexyl)-1-carboxylat-Hydrochlorid, 1-Methyl-4-piperidyldiphenylpropoxyacetat-Hydrochlorid, (3R)-1-Azabicyclo[2.2.2]oct-3-yl-(1S)-1-phenyl-3,4-dihydroisochinolin-2(1H)-carboxylat-succinat, (S)-1-[2-(2,3-Dihydro-5-benzofuranyl)-ethyl]-α,α-diphenyl-3-pyrrolidinacetamid-Hydrobromid, (8R)-3α-Hydroxy-8-isopropyl-1αH,5αH-tropaniumbromid (±Tropat), 8-Butyl-6β,7β-epoxy-3α-[(S)-3-hydroxy-2-phenylpropanoyloxy]-tropaniumbromid, (+)-(S)-N-Methyl-γ-(1-naphthyloxy)-2-thiophenpropylamin, 2-[(1R)-3-(Diisopropylamino)-1-phenylpropyl]-4-(hydroxymethyl)-phenylisobutyrat, 4-(Diethylamino)-but-2-in-1-yl-(2S)-cyclohexyl-(hydroxy)-phenylacetat, Ethyldimethyl-(1-methyl-3,3-diphenylpropyl)-ammoniumbromid, 2-Piperidinoethyl-3-methyl-4-oxo-2-phenyl-4H-chromen-8-carboxylat-Hydrochlorid, Benzyl-(2-chlorethyl)(1-methyl-2-phenoxyethyl)-amin-Hydrochlorid und/oder ein Salz oder ein anderes Salz dieser Wirkstoffe.

Vorzugsweise liegt der Wirkstoff in eine Menge von 1 bis 250 mg pro Dosiseinheit auf den Wirkstoffträgern vor, vorzugsweise in einer Menge, die der Tagesdosis entspricht. Dabei kann der Wirkstoff in Form eines Überzuges auf den Wirkstoffträgern vorliegen, welcher Überzug neben dem Wirkstoff gegebenenfalls zusätzlich ein Bindemittel und gegebenenfalls Trennmittel und/oder Puffersubstanzen aufweisen kann, um die Freisetzung des Wirkstoffes in der gewünschten Weise zu steuern.

Die Wirkstoffträger liegen vorzugsweise in Form von Neutralpellets und/oder kristallinen Substanzen und/oder granulierten oder extrudierten Trägersubstanzen vor und besitzen mit Vorteil eine Teilchengröße von 0,1 bis 3,0 mm, vorzugsweise von 0,2 bis 2,0 mm.

Solche Wirkstoffträger sind im Handel erhältlich und umfassen als Trägermaterial beispielsweise Zucker, Stärke, mikrokristalline Cellulose, Dicalciumphosphat, Natriumchlorid, Citronensäure, Weinsäure, Apfelsäure, Saccharose, Lactose, Sorbitol, Mannitol, Cellulose, Calciumhydrogenphosphat, Natriumcitrat, Tricalciumphosphat, Maisstärke, Weizenstärke, Kartoffelstärke, Reisstärke und/oder Mischungen davon.

Gemäß einer vorteilhaften Ausführungsform der Erfindung enthalten die mit dem Wirkstoff versehenen Wirkstoffträger pro Dosiseinheit 1 bis 250 mg, vorteilhafter Weise 5 bis 100 mg, noch bevorzugter 20 bis 60 mg des Wirkstoffes, 10 bis 500 Gewichtsteile Trägermaterial, 1 bis 100 Gewichtsteile Bindemittel, 1 bis 100 Gewichtsteile Trennmittel und 1 bis 100 Gewichtsteile Puffersubstanzen.

Das Bindemittel der mit dem Wirkstoff versehenen Wirkstoffträger kann aus der Hydroxypropylmethylcellulose, Butylmethacrylat-(2-Dimethylaminoethyl)-methacrylat-Methylmethacrylat-Copolymere, Ethylacrylat-Methylmethacrylat-Copolymere, Methacrylsäure-Methylmethacrylat-Copolymere, Methacrylsäure-Ethylacrylat-Copolymere, Methylacrylat-Methylmethacrylat-Methacrylsäure-Copolymere, Ethylacrylat-Methylmethacrylat-Trimethylammoniumethylmethacrylatchlorid-Copolymere, Ethylcellulose, Natriumcarboxymethylcellulose, Methylcellulose, Hydroxyethylcellulose, Methylethylcellulose, Hydroxyethylmethylpropylcellulose, Polyvinylpyrrolidon, Polyvinalacetat-Vinylpyrrolidon-Vinylacetat-Copolymere, Polyethylenglykol, Gelatine, Maisstärke, Weizenstärke, Reisstärke und Kartoffelstärke und Mischungen davon umfassenden Gruppe ausgewählt werden.

Als Trennmittel kann der mit dem Wirkstoff versehene Wirkstoffträger Talkum, Stearinsäure und deren Salze, Mono-, Di-, Triglyceride mit gerad- und/oder verzweigtkettigen Fettsäuren, Fettalkohole, hochdisperses Siliciumdioxid, gefälltes Siliciumdioxid, Aluminiumoxid, hydriertes Rizinusöl, und Macrogol (Polyethylenglykol) und Mischungen davon aufweisen.

Als Puffersubstanzen zur Verhinderung von möglichen pH-Verschiebungen im Darmbereich der mit dem Wirkstoff versehenen Wirkstoffträger kann man Natriumhydroxid, Citronensäure, Weinsäure, Phosphorsäure, Ascorbinsaure, Bernsteinsäure, Adipinsäure, Fumarsäure und deren pharmazeutisch annehmbare Salze und Mischungen davon einsetzen.

Die auf der inneren Schicht zur Steuerung der Wirkstofffreisetzung angeordnete magensaftresistente Überzugsschicht ist vorzugsweise aus der Ethylcellulose, Celluloseacetatphthalat, Celluloseacetatsuccinat, Schellack, Hydroxypropylmethylcelluloseacetatsuccinat, Celluloseacetat, Celluloseacetatpropionat, Hydroxypropylmethylcellulosephthalat, Polyvinylacetatphthalat, Celluloseacetatbutyrat, Butylmethacrylat-2-Dimethylaminoethyl-methacrylat-Methylmethacrylat-Copolymer, Ethylacrylat-Methylmethacrylat-Copolymer, Methacrylsäure-Methylmethacrylat-Copolymer, Methacrylsäure-Ethylacrylat-Copolymer, Methacrylsäure-Methylmethacrylat-Copolymer, Methylacrylat-Methylmethacrylat-Methacrylsäure-Copolymer, Ethylacrylat-Methylmethacrylat-Trimethylamminoethylmethacrylatchlorid-Copolymer und/oder Mischungen davon umfassenden Gruppe gebildet.

Bei einer Art der Einnahme der Wirkstoffträger, bei welcher diese direkt mit dem Speichel im Mundraum in Kontakt kommen, kann es aufgrund der pH-Verhältnisse zu einer Auflösung der magensaftresistenten Schicht kommen, wobei dann bereits Wirkstoff freigesetzt wird. Um dies zu verhindern ist es erfindungsgemäß bevorzugt, die mit dem Wirkstoff, der Diffusionsschicht und gegebenenfalls dem magensaftresistenten Überzug versehenen Wirkstoffträger mit einer speichelresistenten Außenschicht zu versehen, die vorzugsweise aus Ethylcellulose, Celluloseacetatphthalat, Celluloseacetatsuccinat, Schellack, Hydroxypropylmethylcelluloseacetatsuccinat, Celluloseacetat, Celluloseacetatpropionat, Hydroxypropylmethylcellulosephthalat, Polyvinylacetat, Polyvinylacetatphthalat, Polyvinylalkohol-Polyethylenglykol-Copolymer, Celluloseacetatbutyrat, Butylmethacrylat-2-Dimethylaminoethyl-methacrylat-Methylmethacrylat-Copolymer, Ethylacrylat-Methylmethacrylat-Copolymer, Methacrylsäure-Methylmethacrylat-Copolymer, Methacrylsäure-Ethylacrylat-Copolymer, Methacrylsäure-Methylmethacrylat-Copolymer, Methylacrylat-Methylmethacrylat-Methacrylsäure-Copolymer, Ethylacrylat-Methylmethacrylat-Trimethylamminoethylmethacrylatchlorid-Copolymer und/oder Mischungen davon gebildet wird.

Gewünschtenfalls kann die speichelresistente Außenschicht Aromastoffe, Geschmacksstoffe und/oder Süßungsmittel enthalten.

Gemäß einer weiter bevorzugten Ausführungsform der Erfindung sind die mit dem Wirkstoff, den Diffusionsschichten, der magensaftresistenten Überzugsschicht und gegebenenfalls der speichelresistenten Außenschicht versehenen Wirkstoffträger unter Verwendung üblicher Hilfsstoffe zu Tabletten verpresst oder in Kapseln aus Gelatine, Cellulose, Stärke oder Stärkederivaten abgefüllt oder liegen in Form von Flüssigkeiten oder halbfesten oder festen Zubereitungen für die Herstellung einer Suspension oder eines Suspensionsgels vor.

Die erfindungsgemäße pharmazeutische Zubereitung kann unter Anwendung an sich bekannter Verfahrensweisen hergestellt werden, beispielsweise dadurch, daß man die beispielsweise in Form von handelsüblichen Pellets vorliegenden Wirkstoffträger mit einer Lösung oder Suspension, die den Wirkstoff, das Bindemittel, das Trennmittel und gegebenenfalls Puffersubstanzen enthält, beschichtet, dann das in dieser Weise erhaltene Zwischenprodukt nacheinander mit mindestens zwei Diffusionsschichten versieht, deren Durchlässigkeit für den diffundierenden Wirkstoff von innen nach außen abnimmt, die magensaftresistente Überzugsschicht aufträgt und gegebenenfalls eine speichelresistente Außenschicht vorsieht.

Es hat sich gezeigt, daß die erfindungsgemäße Steuerung der Wirkstofffreisetzung über die innere Schicht aus mindestens zwei Diffusionsschichten, deren Durchlässigkeit für den diffundierenden Wirkstoff von innen nach außen abnimmt, den überraschenden Vorteil besitzt, daß im Vergleich zu anderen bekannten Formulierungen ein geringerer Anstieg der Wirkstofffreisetzung erfolgt und somit die gewünschten niedrigeren Blutspiegel des Wirkstoffes erhalten werden, wobei sich im Vergleich zu der Lehre der US 2004/0142035 A1 überraschenderweise ein noch günstigeres Wirkstofffreisetzungsverhalten dadurch ergibt, daß man zwei Schichten mit unterschiedlicher Diffusionsdurchlässigkeit aufbringt anstelle einer Schicht, welche die beiden Materialien mit unterschiedlichen Diffusionsverhalten in Form einer Mischung enthält.

Dieser Sachverhalt sei durch die folgenden Beispiele und Vergleichsbeispiele näher erläutert.

Die erfindungsgemäße pharmazeutische Zubereitung kann vorzugsweise in 2 Darreichungsformen vorliegen:

1. Eine Kapsel, die mit den mit dem Wirkstoff, den Diffusionsschichten, der magensaftresistenten Überzugsschicht und der gegebenenfalls speichelresistenten Außenschicht versehenen Wirkstoffträgern befüllt ist;

2. eine Tablette, die durch Verpressen der mit dem Wirkstoff, den Diffusionsschichten, der magensaftresistenten Überzugsschicht und gegebenenfalls der speichelresistenten Außenschicht versehenen Wirkstoffträger unter Verwendung üblicher Hilfsstoffe erhalten worden ist.

Die Tablette kann teilbar sein und kann entweder mit Flüssigkeit geschluckt, mit geeigneter zerkleinerter Nahrung eingenommen werden oder aber auch gelutscht oder im Mund zerfallen gelassen werden, ohne daß dadurch die angestrebte Wirkstofffreisetzung im Dünndarm beeinträchtigt wird, weil die Tablette bei diesen Einnahmeformen in die einzelnen oder agglomerierten Wirkstoffträger zerfällt, welche Wirkstoffträger jeweils den Wirkstoff, die Diffusionsschichten und die magensaftresistente Überzugsschicht aufweisen, wodurch das angestrebte Wirkstofffreisetzungsprofil sichergestellt wird.

Bei Anwesenheit einer speichelresistenten Außenschicht wird bei den Einnahmeformen der pharmazeutischen Zubereitung erreicht, daß es aufgrund der pH-Verhältnisse im Mundraum nicht zu einer Beeinträchtigung der magensaftresistenten Schicht kommt und somit der Wirkstoff auch nicht im Mundraum sondern bestimmungsgemäß erst im Dünndarm freigesetzt wird.

**BEISPIEL 1 Charge: 050046/2**

**Erfindungsgemäße pharmazeutische Zubereitung in Form von Pellets mit einem Wirkstoffgehalt von 45 mg (1R,3r,5S)-3-[(Hydroxydiphenylacetyl)-oxy]-spiro[8-azoniabicyclo[3.2.1]octan-8,1'-pyrrolidinium]-chlorid als Wirkstoff je Dosierungseinheit**

Die Bestandteile für die Herstellung der mit dem Wirkstoff versehenen Wirkstoffträger sind in der folgenden Tabelle 1 aufgeführt.

| Tabelle 1 | | |
|---|---|---|
| **Rezeptur, Auftragen des Wirkstoffes** | | |
| Nr. | Ausgangsstoff | pro Dosis mg |
| 1 | Wirkstoff* | 45,000 |
| 2 | Neutralpellets | 100,000 |
| 3 | Hydroxypropylmethylcellulose (Hypromellose) | 4,500 |
| 4 | Talkum | 4,500 |
| 5 | Polyethylenglykol 6.000 (Macrogol 6.000) | 0,450 |
| | Gesamtmenge pro Dosis | 154,450 |
| *(1R,3r,5S)-3-[(Hydroxydiphenylacetyl)-oxy]-spiro[8-azoniabicyclo[3.2.1]octan-8, 1'-pyrrolidinium]-chlorid | | |

Bei den Neutralpellets handelt es sich um Zucker-Stärke-Pellets mit einer Teilchengröße im Bereich von 0,1 bis 3,0 mm. Der Wirkstoff, die Hydroxypropylmethylcellulose und Macrogol 6.000 werden in Wasser gelöst und Talkum in der Lösung suspendiert. Die Suspension wird in einem Wirbelschichtgranulator auf die Wirkstoffträger kontinuierlich aufgesprüht und gleichzeitig bei einer Zuluft-Temperatur von 30°C bis 80°C getrocknet.

Anschließend wird die Diffusionsschicht 1 auf die in dieser Weise mit dem Wirkstoff versehenen Wirkstoffträgern aufgebracht unter Verwendung der in der nachfolgenden Tabelle 2 angegebenen Bestandteile:

| Tabelle 2 | | |
|---|---|---|
| **Rezeptur, Auftragen einer Diffusionsschicht zur optimierten Wirkstofffreigabe** | | |
| Rezeptur Diffusionsschicht 1 | | |
| Nr. | Ausgangsstoff | pro Dosis mg |
| 1 | Wirkstoffpellets (gemäß Tabelle 1) | 154,450 |
| 2 | Polyvinylacetat stabilisiert mit Polyvinylpyrrolidon und Natriumlaurylsulfat (Kollicoat SR 30 D) | 9,000 |
| 3 | Polyvinylalkohol-Polyethylenglycol-Copolymer (Kollicoat IR) | 1,8000 |
| 4 | Propylenglycol | 0,900 |
| 5 | Talkum | 0,360 |
| | Gesamtmenge pro Dosis | 166,510 |

Die Diffusionsschicht 1 wird in der Weise hergestellt, daß die unter Verwendung der in der Tabelle 1 angegebenen Bestandteile erhaltenen, mit dem Wirkstoff versehenen Wirkstoffträger mit einer wässrigen Suspension aus Polyvinylacetat als Matrixmaterial und Polyvinylalkohol-Polyethylenglycol-Copolymer als Porenbildner, Propylenglycol als Weichmacher und Talkum als Trennmittel besprüht werden.

Anschließend wird die Diffusionsschicht 2 aufgebracht unter Verwendung der in der nachfolgenden Tabelle 3 angegebenen Bestandteile:

| Tabelle 3 | | |
|---|---|---|
| Rezeptur Diffusionsschicht 2 | | |
| Nr. | Ausgangsstoff | pro Dosis mg |
| 1 | Wirkstoffpellets mit Diffusionsschicht 1 (gemäß Tabelle 2) | 166,510 |
| 2 | Polyvinylacetat stabilisiert mit Polyvinylpyrrolidon und Natriumlaurylsulfat (Kollicoat SR 30 D) | 9,000 |

(fortgesetzt)

| Tabelle 3 | | |
|---|---|---|
| Rezeptur Diffusionsschicht 2 | | |
| Nr. | Ausgangsstoff | pro Dosis mg |
| 3 | Polyvinylalkohol-Polyethylenglycol-Copolymer (Kollicoat IR) | 0,405 |
| 4 | Propylenglycol | 0,900 |
| 5 | Talkum | 0,360 |
| | Gesamtmenge pro Dosis | 177,175 |

Die unter der Verwendung der Bestandteile der Tabelle 2 erhaltenen mit dem Wirkstoff und der Diffusionsschicht 1 versehenen Wirkstoffpellets werden mit einer wässrigen Suspension von Polyvinylacetat als Matrixmaterial und Polyvinylalkohol-Polyethylenglycol-Copolymer als Porenbildner, Propylenglycol als Weichmacher und Talkum als Trennmittel besprüht. Wie zu erkennen ist, enthält das Material zur Ausbildung der Diffusionsschicht 2 eine geringere Menge des Porenbildners Polyvinylalkohol-Polyethylenglycol-Copolymer und führt demzufolge zu einer Diffusionsschicht mit einer geringeren Durchlässigkeit für den diffundierenden Wirkstoff (1R,3r,5S)-3-[(Hydroxydiphenylacetyl)-oxy]-spiro[8-azoniabicyclo[3.2.1]octan-8,1'-pyrrolidinium]-chlorid.

Die in dieser Weise mit den beiden Diffusionsschichten versehenen Wirkstoffträger werden anschließend mit einem magensaftresistenten Überzug versehen unter Anwendung der in der folgenden Tabelle 4 angegebenen Rezeptur:

| Tabelle 4 | | |
|---|---|---|
| **Rezeptur, Auftragen eines magensaftresistenten Überzuges** | | |
| Nr. | Ausgangsstoff | pro Dosis mg |
| 1 | Wirkstoffpellets mit Diffusionsschichten 1 und 2 (gemäß Tabelle 3) eine Dosis enthält 45 mg Wirkstoff | 177,175 |
| 2 | Methacrylsäure-Ethylacrylat-Copolymer (Kollicoat MAE 30 DP) | 28,000 |
| 3 | Talkum | 12, 600 |
| 4 | Propylenglycol | 4,200 |
| 5 | Natriumcarboxymethycellulose (Tylopur C30 G1) | 0,720 |
| | Gesamtmenge pro Dosis | 222,695 |

Die mit den beiden Diffusionsschichten 1 und 2 erhaltenen Wirkstoffpellets werden dann unter Verwendung der in der obigen Tabelle 4 angegebenen Rezeptur mit der magensaftresistenten Überzugsschicht versehen, wobei eine wäßrige Suspension aus Methacrylsäure-Ethylacrylat-Copolymer als Matrixmaterial, Propylenglykol als Weichmacher, Natriumcarboxymethyl-cellulose als Bindemittel und Talkum als Trennmittel auf die mit den beiden Diffusionsschichten 1 und 2 versehenen Wirkstoffpellets kontinuierlich aufgesprüht und gleichzeitig bei einer Zulufttemperatur von 35 bis 80°C getrocknet wird, wobei die erfindungsgemäße pharmazeutische Zubereitung in Form der mit dem Wirkstoff versehenen Wirkstoffpellets erhalten wird.

### BEISPIEL 2 (Vergleich) Charge: 050047

Zum Vergleich werden unter Verwendung der gleichen Bestandteile und der gleichen Mengenverhältnisse mit Wirkstoff versehene Wirkstoffträger hergestellt, welche die Bestandteile der erfindungsgemäßen Diffusionsschichten 1 und 2 nicht in getrennten Schichten, sondern in einer einzigen Diffusionsschicht enthalten.

Hierzu werden unter Verwendung der in der Tabelle 1 des Beispiels 1 angegebenen Bestandteile mit dem Wirkstoff (1R,3r,5S)-3-[(Hydroxydiphenylacetyl)-oxy]-spiro[8-azoniabicyclo[3.2.1]octan-8,1'-pyrrolidinium]-chlorid versehene Wirkstoffträger hergestellt und mit einer Diffusionsschicht versehen unter Verwendung der in der nachfolgenden Tabelle 5 aufgeführten Bestandteile:

| Tabelle 5 | | |
|---|---|---|
| Rezeptur Diffusionsschicht 1+2 aufgetragen als Mischung | | |
| Nr. | Ausgangsstoff | pro Dosis mg |
| 1 | Wirkstoffpellets (gemäß Tabelle 1) | 154,450 |
| 2 | Polyvinylacetat stabilisiert mit Polyvinylpyrrolidon und Natriumlaurylsulfat (Kollicoat SR 30 D) | 18,000 |
| 3 | Polyvinylalkohol-Polyethylenglycol-Copolymer (Kollicoat IR) | 2,205 |
| 4 | Propylenglycol | 1,800 |
| 5 | Talkum | 0, 720 |
| | Gesamtmenge pro Dosis | 177,175 |

Auf die in dieser Weise erhaltenen mit der Diffusionsschicht 1+2, aufgetragen als Mischung, versehenen Wirkstoffpellets bringt man dann nach der Verfahrensweise des Beispiels 1 und unter Verwendung der in der Tabelle 4 des Beispiels 1 angegebenen Bestandteile einen magensaftresistenten Überzug auf und erhält Wirkstoffpellets der in der nachfolgenden Tabelle 6 angegebenen Zusammensetzung:

| Tabelle 6 | | |
|---|---|---|
| Nr. | Ausgangsstoff | pro Dosis mg |
| 1 | Wirkstoffpellets mit Diffusionsschicht 1+2 (gemäß Tabelle 5) eine Dosis enthält 45 mg Wirkstoff | 177,175 |
| 2 | Methacrylsäure-Ethylacrylat-Copolymer (Kollicoat MAE 30 DP) | 28,000 |
| 3 | Talkum | 12,600 |
| 4 | Propylenglycol | 4,200 |
| 5 | Natriumcarboxymethylcellulose (Tylopur C30 G1) | 0,720 |
| | Gesamtmenge pro Dosis | 222,695 |

## BEISPIEL 3 (Vergleich) Charge: 050046/1

Als weiteres Vergleichsbeispiel bereitet man lediglich mit dem Wirkstoff (1R,3r,5S)-3-[(Hydroxydiphenylacetyl)-oxy]-spiro[8-azoniabicyclo[3.2.1]octan-8,1'-pyrrolidinium]-chlorid versehene Wirkstoffträger, die weder eine Diffusionsschicht noch einen magensaftresistenten Überzug aufweisen, und zwar nach der Verfahrensweise des Beispiels 1 und unter Verwendung der in der Tabelle 1 angegebenen Bestandteile, so daß man mit dem Wirkstoff (1R,3r,5S)-3-[(Hydroxydiphenylacetyl)-oxy]-spiro[8-azoniabicyclo[3.2.1]octan-8,1'-pyrrolidinium]-chlorid versehene Wirkstoffträger der in der folgenden Tabelle 7 angegebenen Zusammensetzung erhält.

| Tabelle 7 | | |
|---|---|---|
| Nr. | Ausgangsstoff | pro Dosis mg |
| 1 | Wirkstoff | 45,000 |
| 2 | Neutralpellets | 100,000 |
| 3 | Hydroxypropylmethylcellulose (Hypromellose) | 4,5000 |
| 4 | Talkum | 4,5000 |
| 5 | Polyethylenglykol 6.000 (Macrogol 6.000) | 0,450 |
| | Gesamtmenge pro Dosis | 154,450 |

**BEISPIEL 4 (Vergleich) Charge: 050048**

Dieses Vergleichsbeispiel betrifft mit dem Wirkstoff (1R,3r,5S)-3-[(Hydroxydiphenylacetyl)-oxy]-spiro[8-azoniabicyclo[3.2.1]octan-8,1'-pyrrolidinium]-chlorid versehene Wirkstoffträger, die lediglich mit einer Diffusionsschicht, und zwar der Diffusionsschicht 1 des Beispiels 1 der vorliegenden Erfindung, und der magensaftresistenten Überzugsschicht versehen sind.

Man geht aus von mit dem Wirkstoff versehenen Wirkstoffpellets, die erhalten worden sind nach der Verfahrensweise des Beispiels 1 unter Verwendung der in der Tabelle 1 angegebenen Bestandteile.

Diese Wirkstoffträger werden nur mit der Diffusionsschicht 1 versehen unter Verwendung der Bestandteile und der Verfahrensweise, die für die Ausbildung der Diffusionsschicht 1 in Beispiel 1 unter Bezugnahme auf die Tabelle 2 angegeben sind.

Nach dem Aufbringen einer magensaftresistenten Überzugsschicht nach der Verfahrensweise des Beispiels 1 unter Verwendung der in der Tabelle 4 angegebenen Bestandteile erhält man mit dem Wirkstoff versehene Wirkstoffpellets der in der folgenden Tabelle 8 angegebenen Zusammensetzung:

| Tabelle 8 | | |
|---|---|---|
| Nr. | Ausgangsstoff | pro Dosis mg |
| 1 | Wirkstoffpellets mit Diffusionsschicht 1 eine Dosis enthält 45 mg Wirkstoff | 166,510 |
| 2 | Methacrylsäure-Ethylacrylat-Copolymer (Kollicoat MAE 30 DP) | 28,000 |
| 3 | Talkum | 12,600 |
| 4 | Propylenglycol | 4,200 |
| 5 | Natriumcarboxymethylcellulose (Tylopur C30 G1) | 0,720 |
| | Gesamtmenge pro Dosis | 212,030 |

**BEISPIEL 5 (Vergleich) Charge: 050049**

Dieses Vergleichsbeispiel betrifft mit dem Wirkstoff (1R,3r,5S)-3-[(Hydrox diphenylacetyl)-oxy]-spiro[8-azoniabicyclo[3.2.1]octan-8,1'-pyrrolidinium]chlorid versehene Wirkstoffträger, die lediglich mit der Diffusionsschicht 2 der erfindungsgemäßen pharmazeutischen Zubereitung gemäß Beispiel 1 und der magensaftresistenten Überzugsschicht versehen sind.

Hierzu geht man aus von den mit dem Wirkstoff versehenen Wirkstoffträgern, die unter Verwendung der in der Tabelle 1 des Beispiels 1 angegebenen Bestandteile nach der dort beschriebenen Verfahrensweise hergestellt worden sind.

Auf diese mit dem Wirkstoff versehenen Wirkstoffpellets bringt man die Diffusionsschicht 2 auf unter Verwendung der in der nachfolgenden Tabelle 9 angegebenen Bestandteile und unter Anwendung der Verfahrensweise, die im Zusammenhang mit der Tabelle 3 in Beispiel 1 angegeben ist.

| Tabelle 9 | | |
|---|---|---|
| Nr. | Ausgangsstoff | pro Dosis mg |
| 1 | Wirkstoffpellets (gemäß Tabelle 1) | 154,450 |
| 2 | Polyvinylacetat stabilisiert mit Polyvinylpyrrolidon und Natriumlaurylsulfat (Kollicoat SR 30D) | 9,000 |
| 3 | Polyvinylalkohol-Polyethylenglycol-Copolymer (Kollicoat IR) | 0,405 |
| 4 | Propylenglycol | 0,900 |
| 5 | Talkum | 0,360 |
| | Gesamtmenge pro Dosis | 165,115 |

Nach dem Aufbringen einer magensaftresistenten Überzugsschicht nach der Verfahrensweise des Beispiels 1 unter Verwendung der in der Tabelle 4 angegebenen Bestandteile erhält man Wirkstoffpellets mit der Diffusionsschicht 2 der in der folgenden Tabelle 10 angegebenen Zusammensetzung.

| Tabelle 10 | | |
|---|---|---|
| **Rezeptur, Auftragen eines magensaftresistenten Überzuges** | | |
| Nr. | Ausgangsstoff | pro Dosis mg |
| 1 | Wirkstoffpellets mit Diffusionsschicht 2 eine Dosis enthält 45 mg Wirkstoff | 165,115 |
| 2 | Methacrylsäure-Ethylacrylat-Copolymer (Kollicoat MAE 30 DP) | 28,000 |
| 3 | Talkum | 12,600 |
| 4 | Propylenglycol | 4,200 |
| 5 | Natriumcarboxymethylcellulose (Tylopur C30 G1) | 0,720 |
| | Gesamtmenge pro Dosis | 210,635 |

**BEISPIEL 6**

**Untersuchung zur in vitro-Freisetzung des Wirkstoffes (1R,3r,5S)-3-[(Hydroxydiphenylacetyl)-oxy]-spiro [8-azoniabicyclo[3.2.1]octan-8,1'-pyrrolidinium]-chlorid aus den Wirkstoffpellets, die gemäß den obigen Beispielen 1 bis 5 erhalten worden sind.**

Zur Untersuchung der Wirkstofffreisetzung in Abhängigkeit von der Zeit wird die Vorschrift der US Pharmacopoe zur Bestimmung der Freisetzung von Produkten mit magensaftresistentem Überzug Nr. 724 Methode A wie folgt angewandt:

**Säurestufe**

| | |
|---|---|
| Vorrichtung: | Flügelrührer |
| Drehgeschwindigkeit: | 200 min$^{-1}$ |
| Temperatur: | 37 $\pm$ 0,5°C |
| Lösungsmedium: | 750 ml 0,1 M Chlorwasserstoffsäure |

Man bringt eine Dosis der nach den obigen Beispielen hergestellten Wirkstoffpellets in die Vorrichtung ein, bedeckt das Gefäß und betreibt den Flügelrührer während 2 Stunden. Dann entnimmt man eine Probe zur Bestimmung des Wirkstoffes (1R,3r,5S)-3-[(Hydroxydiphenylacetyl)-oxy]-spiro[8-azoniabicyclo[3.2.1]octan-8,1'-pyrrolidinium]-chlorid.

**Pufferstufe**

250 ml einer auf 37 $\pm$ 0,5°C temperierten 0,20 M dreibasischen Natriumphosphat-Lösung werden zu der Säurestufe (750 ml 0,1M Chlorwasserstoffsäure) zugesetzt. Man stellt den pH-Wert erforderlichenfalls mit 2 N Chlorwasserstoffsäure- oder 2 N Natriumhydroxid-Lösung auf einen Wert von 6,8 $\pm$ 0,05 ein. Man betreibt die Vorrichtung während 2 Stunden und entnimmt dann nach 15, 30, 45, 60, 75, 90, 105 und 120 Minuten Proben, in denen der Wirkstoff durch HPLC bestimmt wird.

**Referenzlösung:**

0,045 mg/ml (1R,3r,5S)-3-[(Hydroxydiphenylacetyl)-oxy]-spiro[8-azoniabicyclo-[3.2.1]octan-8,1'-pyrrolidinium]-chlorid RS in 0,1 N Chlorwasserstoffsäure.

**Bedingungen der HPLC-Chromatographie**

Die Chromatographie erfolgt unter Verwendung:

- einer Säule aus rostfreiem Stahl mit einer Länge von 0,125 m und einem Innendurchmesser von 4 mm, die mit Octadecylsilylgel für chromatographische Zwecke (5 μm) beschickt ist, das heißt Nucleosil 100-5 C18;
- als mobile Phase verwendet man eine Mischung aus 65 Volumen gereinigtem Wasser, welches 2,202 g/l Natriumheptansulfonat und 0,4 ml/l Triethylamin enthält und mit konzentrierter Phosphorsäure auf einen pH-Wert von 2,5 eingestellt worden ist, und 35 Volumen Acetonitril.
- Als Detektor verwendet man ein auf 210 nm eingestelltes Spektrophotometer (DAD 210.10, 400.100 nm);
- man hält die Temperatur der Säule bei 40°C.
- man injiziert 25 μl der Probeflüssigkeit und bewirkt die Chromatographie während etwa 4,5 Minuten.

Man berechnet die Konzentration des gelösten (1R,3r,5S)-3-[(Hydroxydiphenylacetyl)-oxy]-spiro[8-azoniabicyclo-[3.2.1]octan-8,1'-pyrrolidinium]-chlorids in Prozent unter Verwendung der folgenden Gleichung:

$$Y= CR_{(w)} \cdot AT_{(w)} \cdot 100/AR_{(w)} \cdot CT_{(w)}$$

worin

Y:          gelöster Wirkstoff (1R,3r,5S)-3-[(Hydroxydiphenylacetyl)-oxy]-spiro-[8-azoniabicyclo-[3.2.1]octan-8,1'-pyrrolidinium]-chlorid (% des angegebenen Gehalts)
$CT_{(w)}$:     nominale Konzentration des Wirkstoffs in der Testlösung (0,045 mg/ml)
$CR_{(w)}$:     Konzentration des Wirkstoffs in der Referenzlösung
$AT_{(w)}$:     Fläche Wirkstoff-Peak der Testlösung
$AR_{(w)}$:     Fläche Referenzsubstanz-Peak in der Referenzlösung

Die bei dieser Untersuchung der Wirkstoffpellets der Beispiele 1 bis 5 erhaltenen Ergebnisse der Wirkstofffreisetzung sind in der nachfolgenden Tabelle 11 zusammengestellt:

**PU040014 W 45 Tabletten MOF**

**Wirkstoffpellets und magensaftresistente Pellets mit unterschiedlichem Auftrag der Diffusionsschichten**

EP 1 880 718 B1

**Tabelle 11**

**Ergebnisse der Freisetzung des Wirkstoffs***

| Prüfzeitpunkt/ | Bps. 3 (Ref.)<br>Wirkstoffpellets ohne<br>Diffusionsschicht | Bsp. 1 (Erfindung)<br>Diffusionsschicht 1+2<br>nacheinander aufgetragen | Bsp. 2 (Vergl.)<br>Diffusionsschicht 1+2 als<br>Mischung aufgetragen | Bsp. 4 (Vergl.)<br>Diffusionsschicht 1 allein<br>aufgetragen | Bsp. 5 (Vergl.)<br>Diffusionsschicht 2 allein<br>aufgetragen |
|---|---|---|---|---|---|
| Charge | 050046/1 | 050046/2 | 050047 | 050048 | 050049 |
| 0 | 0,00 | 0 | 0 | 0 | 0 |
| 15 | 98,02 | 0,76 | 73,94 | 101,02 | 7,02 |
| 30 | 98,30 | 8,14 | 91,12 | 101,24 | 34,27 |
| 45 | 98,39 | 29,89 | 97,84 | 101,03 | 67,08 |
| 60 | 98,47 | 51,13 | 98,75 | 101,20 | 81,80 |
| 75 | 98,65 | 65,47 | 99,59 | 101,25 | 88,84 |
| 90 | 98,56 | 74,35 | 99,45 | 101,30 | 92,39 |
| 105 | 98,49 | 80,03 | 99,99 | 101,35 | 94,63 |
| 120 | 98,68 | **84,09** | 99,89 | **101,10** | **95,87** |
| *(1R,3r,5S)-3-[(Hydroxydiphenylacetyl)-oxy]-spiro[8-azoniabicyclo-[3.2.1]octan-8,1'-pyrrolidinium]-chlorid | | | | | |

12

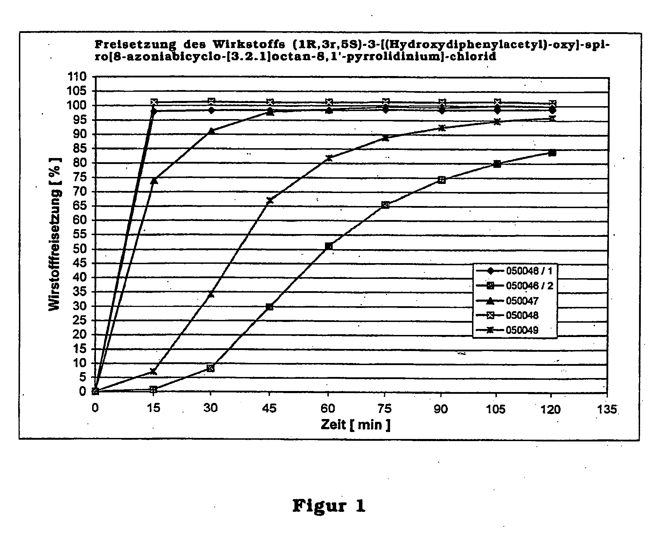

In der **Figur 1** sind die Ergebnisse der obigen Tabelle 11 graphisch dargestellt, und zwar durch Auftragung der prozentualen Wirkstofffreisetzung in Abhängigkeit von der Zeit.

Wie aus der Figur 1 hervorgeht, zeigt die erfindungsgemäße pharmazeutische Zubereitung (050046/2) den besten und erfindungsgemäß gewünschten Verlauf der in vitro-Wirkstofffreisetzung mit einem langsamen Anstieg und anschließender quasi konstanter Wirkstofffreisetzung, während die Vergleichspräparate, die keine Diffusionsschicht aufweisen (050046/1) beziehungsweise jeweils nur eine Diffusionsschicht 1 oder 2 aufweisen (050049 beziehungsweise 050048) oder die Wirkstoffpellets gemäß Beispiel 2, bei denen die Bestandteile der beiden erfindungsgemäßen Diffusionsschichten 1 und 2 in einer einzigen Diffusionsschicht enthalten sind (050047), einen schnellen Anstieg der Kurven und somit eine unerwünschte schnelle Wirkstofffreisetzung.

Als überraschend anzusehen ist, daß die erfindungsgemäße pharmazeutische Zubereitung, bei der die innere Schicht aus mindestens zwei Diffusionsschichten ausgebildet ist, deren Durchlässigkeit für den diffundierenden Wirkstoff von innen nach außen abnimmt, einen wesentlich besseren Verlauf der Wirkstofffreisetzung zeigt als die Wirkstoffpellets gemäß Beispiel 2, bei dem die gleichen Bestandteile für die Ausbildung der erfindungsgemäßen beiden Diffusionsschichten in einer einzigen Diffusionsschicht enthalten sind. Auch diese pharmazeutische Zubereitung zeigt eine unerwünscht schnelle Wirkstofffreisetzung zu Beginn, indem annähernd 95% bereits nach 40 Minuten freigesetzt sind, zu einem Zeitpunkt, da die erfindungsgemäße pharmazeutische Zubereitung lediglich eine Wirkstofffreisetzung von etwa 20% ergibt.

Mit der erfindungsgemäßen pharmazeutischen Zubereitung wird es also möglich, im Vergleich zu der einmaligen Verabreichung eines den Wirkstoff (1R,3r,5S)-3-[(Hydroxydiphenylacetyl)-oxy]-spiro[8-azoniabicyclo-[3.2.1]octan-8,1'-pyrrolidinium]-chlorid schnell freisetzenden pharmazeutischen Präparats eine langsamere Anflutung des Wirkstoffes im Plasma bei einem insgesamt niedrigeren Blutspiegel zu erreichen, und damit die Nachteile der herkömmlichen Produkte dieser Art zu überwinden.

**Patentansprüche**

1. Pharmazeutische Zubereitung für die orale Verabreichung mit gesteuerter Wirkstofffreisetzung im Dünndarm, auf der Grundlage von mit mindestens einem Wirkstoff versehenen Wirkstoffträgern, die mit einer inneren Schicht zur Steuerung der Wirkstofffreisetzung und einer darauf angeordneten magensaftresistenten Überzugsschicht versehen sind, **dadurch gekennzeichnet,**

   **daß** die innere Schicht aus mindestens zwei Diffusionsschichten ausgebildet ist, deren Durchlässigkeit für den diffundierenden Wirkstoff von innen nach außen abnimmt, wobei die Diffusionsschichten aus einem im Dünndarmsaft unlöslichen Matrixmaterial gebildet sind, in das die Durchlässigkeit für den diffundierenden Wirkstoff steuernde, im Dünndarmsaft lösliche und/oder quellbare und/oder wasseraufnehmende Porenbildner eingelagert sind,

   und **daß** der Porenbildner der Diffusionsschichten aus der Polyvinylalkohol-Polyethylenglykol-Copolymere, Polyvinylpyrrolidon, Vinylpyrrolidon-Vinylacetat-Copolymere, vernetztes Polyvinylpyrrolidon, mikrokristalline Cellulose, Hydroxypropylcellulose, Natriumcarboxymethylcellulose, Methylcellulose, Hydroxyethylcellulose, Methylethylcellulose, Methylhydroxypropylcellulose, Hydroxypropylmethylcellulose, Hydroxyethylmethylpropylcellulose, Polyethylenglykol. Cellulosepulver, Saccharose, Lactose, Mannitol, Sorbitol und Polysorbat bestehende Gruppe ausgewählt ist,

   und **daß** die Wirkstoffträger als Wirkstoff 4-Diethylamino-2-butinyl-$\alpha$-phenylcyclohexaneglyconat-Hydrochlorid, Ethyldimethyl-(1-methyl-3,3-diphenylpropyl)-ammoniumcarrageenat, (+)-(R-2-[$\alpha$-2-(Diisopropylamino)-ethyl]-benzyl)-p-cresol-tartrat, 8-(Cyclopropylmethyl)-6$\beta$,7$\beta$-epoxy-3$\alpha$-hydroxy-1$\alpha$H,5$\alpha$)-(S)-tropat-bromid, (1R,3r,5S)-3-[(Hydroxydiphenylacetyl)-oxy]-spiro[8-azoniabicyclo[3.2.1]octan-8,1'-pyrrolidinium]-chlorid, 2-Diethylaminoethyl-(bicyclohexyl)-1-carboxylat-Hydrochlorid, 1-Methyl-4-piperidyldiphenylpropoxyacetat-Hydrochlorid, (3R)-1-Azabicyclo[2.2.2]oct-3-yl-(1S)-1-phenyl-3,4-dihydroisochinolin-2(1H)-carboxylat-succinat, (S)-1-[2-(2,3-Dihydro-5-benzofuranyl)-ethyl]-$\alpha$,$\alpha$-diphenyl-3pyrrolidinacetamid-Hydrobromid, (8R)-3$\alpha$-Hydroxy-8-isopropyl-1$\alpha$H,5$\alpha$H-tropaniumbromid ($\pm$Tropat), 8-Butyl-6$\beta$,7$\beta$-epoxy-3$\alpha$-[(S)-3-hydroxy-2-phenylpropanoyloxyl]-tropaniumbromid, (+)-(S)-N-Methyl-(1-naphthyloxy)-2-thio-phenpropylamin, 2-[(1R)-3-(Diisopropylamino)-1phenylpropyll-4-(hydroxymethyl)-phenylisobutyrat, 4-(Diethylamino)-but-2-in-1-yl-(2S)-cyclohexyl-(hydroxy)-phenylacetat, Ethyldimethyl-(1-methyl-3,3-diphenylpropyl)-ammoniumbromid, 2-Piperidinoethyl-3-methyl-4-oxo-2-phenyl-4H-chromen-8-carboxylat-Hydrochlorid, Benzyl-(2-chlorethyl)(1-methyl-2-phenoxyethyl)-amin-Hydrochlorid und/oder ein Salz oder ein anderes Salz dieser Wirkstoffe aufweisen.

2. Pharmazeutische Zubereitung nach Anspruch 1, **dadurch gekennzeichnet, daß** die Diffusionsschichten gleiche oder verschiedenartige Matrixmaterialien umfassen.

3. Pharmazeutische Zubereitung nach mindestens einem der Ansprüche 1 oder 2, **dadurch gekennzeichnet, daß** die Diffusionsschichten einen oder mehrere, gleiche oder verschiedenartige Porenbildner enthalten.

4. Pharmazeutische Zubereitung nach mindestens einem der vorhergehenden Ansprüche, **dadurch gekennzeichnet, daß** die Durchlässigkeit der Diffusionsschichten für den diffundierenden Wirkstoff über die Art, die Menge und/oder die Teilchengröße und/oder die Löslichkeit und/oder die Quellbarkeit und/oder das Wasseraufnehmvermögen der Porenbildner gesteuert wird.

5. Pharmazeutische Zubereitung nach Anspruch 4, **dadurch gekennzeichnet, daß** die innere Schicht zur Steuerung der Wirkstofffreisetzung eine innere und eine äußere Diffusionsschicht umfaßt.

6. Pharmazeutische Zubereitung nach Anspruch 5, **dadurch gekennzeichnet, daß** bei identischem Porenbildnermaterial die Teilchengröße und/oder die Konzentration der Porenbildner in der inneren Diffusionsschicht größer ist als in der äußeren Diffusionsschicht.

7. Pharmazeutische Zubereitung nach Anspruch 6, **dadurch gekennzeichnet, daß** bei im Dünndarmsaft löslichen Porenbildnern das Verhältnis der Porenbildner-Konzentration in der inneren Diffusionsschicht zu der Porenbildner-Konzentration in der äußeren Diffusionsschicht im Bereich von 20:1 bis >1:1, vorzugsweise 10:1 bis 1.1:1 liegt.

8. Pharmazeutische Zubereitung nach mindestens einem der vorhergehenden Ansprüche, **dadurch gekennzeichnet, daß** das Matrixmaterial der Diffusionsschichten aus der Ethylcellulose, Celluloseacetatphthalat, Celluloseacetatsuccinat, Schellack, Hydroxypropylmethylcelluloseacetatsuccinat, Celluloseacetat, Celluloseacetatpropionat, Hydroxypropylmethylcellulosephthalat, Polyvinylacetat, Polyvinylacetatphthalat, Celluloseacetatbutyrat, Butylmethacrylat-(2-Dimethylaminoethyl)-methacrylat-Methylmethacrylat-Copolymere, Ethylacrylat-Methylmethacrylat-Copolymere, Methacrylsäure-Methylmethacrylat-Copolymere, Methacrylsäure-Ethylacrylat-Copolymere, Ethylacrylat-Methylmethacrylat-Trimethylammoniumethylmethacrylatchlorid-Copolymere, Chitosan, Siliconelastomer-Latexsuspensionen hydriertes Rizinusöl, Stearinsäure, Glycerinmonostearat, Glycerindistearat, Glycerindibehenat, Stearylalkohol, weißes Wachs, gelbes Wachs, hydriertes Pflanzenöl und mikrokristallines Wachs umfassenden Gruppe ausgewählt ist.

9. Pharmazeutische Zubereitung nach mindestens einem der vorhergehenden Ansprüche, **dadurch gekennzeichnet, daß** das Matrixmaterial der Diffusionsschichten zusätzlich Weichmacher und Trennmittel enthält.

10. Pharmazeutische Zubereitung nach Anspruch 9, **dadurch gekennzeichnet, daß** das Matrixmaterial der Diffusionsschichten als Weichmacher Polyethylenglykol, Propylenglykol, Triethylcitrat, Triacetin, Acetyltributylcitrat, Polysorbate, 2-Pyrrolidon, Sebacinsäuredibutylester, Stearinsäure, Rizinusöl und/oder mittelkettige Triglyceride, und als Trennmittel Talkum, Stearinsäure und deren Salze, Fettalkohole, Mono-, Di- oder Triglyceride mit gerad- und/oder verzweigtkettigen Fettsäuren, hochdisperses Siliciumdioxid, gefälltes Siliciumdioxid, Aluminiumoxid, Kaolin, Maisstärke, Weizenstärke, Reisstärke, Kartoffelstärke, Titandioxid, Siliconemulsionen und/oder Veegum (eine Magnesium-Aluminium-silikat-Dispersion) enthält.

11. Pharmazeutische Zubereitung nach Anspruch 10, **dadurch gekennzeichnet, daß** der Wirkstoff in einer Menge von 1 bis 250 mg pro Dosiseinheit auf den Wirkstoffträgern vorliegt.

12. Pharmazeutische Zubereitung nach Anspruch 10 oder 11, **dadurch gekennzeichnet, daß** der Wirkstoff in Form eines Überzugs auf den Wirkstoffträgern vorliegt.

13. Pharmazeutische Zubereitung nach mindestens einem der vorhergehenden Ansprüche, **dadurch gekennzeichnet, daß** die Wirkstoffträger in Form von Neutralpellets und/oder kristallinen Substanzen und/oder granulierten oder extrudierten Trägersubstanzen vorliegen.

14. Pharmazeutische Zubereitung nach Anspruch 13, **dadurch gekennzeichnet, daß** die Wirkstoffträger eine Teilchengröße von 0,1 bis 3,0 mm aufweisen.

15. Pharmazeutische Zubereitung nach Anspruch 13 oder 14, **dadurch gekennzeichnet, daß** die Wirkstoffträger als Trägermaterial Zucker, Stärke, mikrokristalline Cellulose, Dicalciumphosphat, Natriumchlorid, Citronensäure, Weinsäure, Apfelsäure, Saccharose, Lactose, Sorbitol, Mannitol, Cellulose, Calciumhydrogenphosphat, Natriumcitrat, Tricalciumphosphat, Maistärke, Weizenstärke, Kartoffelstärke, Reisstärke und/oder Mischungen davon umfassen.

**16.** Pharmazeutische Zubereitung nach mindestens einem der vorhergehenden Ansprüche, **dadurch gekennzeichnet, daß** die mit dem Wirkstoff versehenen Wirkstoffträger an ihrer Oberfläche neben dem Wirkstoff zusätzlich ein Bindemittel und gegebenenfalls Trennmittel und/oder Puffersubstanzen aufweisen.

**17.** Pharmazeutische Zubereitung nach Anspruch 16, **dadurch gekennzeichnet, daß** die mit dem Wirkstoff versehenen Wirkstoffträger pro Dosiseinheit 1 bis 250 mg Wirkstoff, 10 bis 500 Gewichtsteile Trägermaterial, 1 bis 100 Gewichtsteile Bindemittel, 1 bis 100 Gewichtsteile Trennmittel und 1 bis 100 Gewichtsteile Puffersubstanz enthalten.

**18.** Pharmazeutische Zubereitung nach den Ansprüchen 16 und 17, **dadurch gekennzeichnet, daß** das Bindemittel der mit dem Wirkstoff versehenen Wirkstoffträger aus der Hydroxypropylmethylcellulose, Butylmethacrylat-(2-Dimethylaminoethyl)-methacrylat-Methylmethacrylat-Copolymere, Ethylacrylat-Methylmethacrylat-Copolymere, Methacrylsäure-Methylmethacrylat-Copolymere, Methacrylsäure-Ethylacrylat-Copolymere, Methylacrylat-Methylmethacrylat-Methacrylsäure-Copolymere, Ethylacrylat-Methylmethacrylat-Trimethylammoniumethylmethacrylatchlorid-Copolymere, Ethylcellulose, Natriumcarboxymethylcellulose, Methylcellulose, Hydroxyethylcellulose, Methylethylcellulose, Hydroxyethylmethylpropylcellulose, Polyvinylpyrrolidon, Polyvinylacetat-Vinylpyrrolidon-Vinylacetat-Copolymere, Polyethylenglykol, Gelatine, Maisstärke, Weizenstärke, Reisstärke und Kartoffelstärke und Mischungen davon umfassenden Gruppe ausgewählt ist.

**19.** Pharmazeutische Zubereitung nach den Ansprüchen 16 bis 18, **dadurch gekennzeichnet, daß** das Trennmittel der mit dem Wirkstoff versehenen Wirkstoffträger aus der Talkum, Stearinsäure und deren Salze, Mono-, Di-, Triglyceride mit gerad- und/oder verzweigtkettigen Fettsäuren, Fettalkohole, hochdisperses Siliciumdioxid, gefälltes Siliciumdioxid, Aluminiumoxid, hydriertes Rizinusöl, und Macrogol (Polyethylenglykol) und Mischungen davon umfassenden Gruppe ausgewählt ist.

**20.** Pharmazeutische Zubereitung nach den Ansprüchen 16 bis 19, **dadurch gekennzeichnet, daß** die Puffersubstanzen der mit dem Wirkstoff versehenen Wirkstoffträger aus der Natriumhydroxid, Citronensäure, Weinsäure, Phosphorsäure, Ascorbinsaure, Bernsteinsäure, Adipinsäure, Fumarsäure und deren pharmazeutisch annehmbare Salze und Mischungen davon umfassenden Gruppe ausgewählt sind.

**21.** Pharmazeutische Zubereitung nach mindestens einem der vorhergehenden Ansprüche, **dadurch gekennzeichnet, daß** die auf dem mit dem Wirkstoff und den Diffusionsschichten versehenen Wirkstoffträger angeordnete magensaftresistente Überzugschicht aus der Ethylcellulose, Celluloseacetatphthalat Celluloseacetatsuccinat, Schellack, Hydroxypropylmethylcelluloseacetatsuccinat, Celluloseacetat, Celluloseacetatpropionat, Hydroxypropylmethylcellulosephthalat, Polyvinylacetatphthalat, Celluloseacetatbutyrat, Butylmethacrylat-2-Dimethylaminoethylmethacrylat-Methylmethacrylat-Copolymer, Ethylacrylat-Methylmethacrylat-Copolymer, Methacrylsäure-Methylmethacrylat-Copolymer, Methacrylsäure-Ethylacrylat-Copolymer, Methacrylsäure-Methylmethacrylat-Copolymer, Methylacrylat-Methylmethacrylat-Methacrylsäure-Copolymer, Ethylacrylat-Methylmethacrylat-Trimethylamminoethylmethacrylatchlorid-Copolymer und/oder Mischungen davon umfassenden Gruppe gebildet ist.

**22.** Pharmazeutische Zubereitung nach mindestens einem der vorhergehenden Ansprüche, **dadurch gekennzeichnet, daß** die mit dem Wirkstoff, den Diffusionsschichten und gegebenenfalls dem magensaftresistenten Überzug versehenen Wirkstoffträger mit einer speichelresistenten Außenschicht versehen sind.

**23.** Pharmazeutische Zubereitung nach Anspruch 22, **dadurch gekennzeichnet, daß** die mit dem Wirkstoff, den Diffusionsschichten und gegebenenfalls dem magensaftresistenten Überzug versehenen Wirkstoffträger mit einer speichelresistenten Außenschicht aus der Ethylcellulose, Celluloseacetatphthalat, Celluloseacetatsuccinat, Schellack, Hydroxypropylmethylcelluloseacetatsuccinat, Celluloseacetat, Celluloseacetatpropionat, Hydroxypropylmethylcellulosephthalat, Polyvinylacetat, Polyvinylacetatphthalat, Polyvinylalkohol-Polyethylenglykol-Copolymer, Celluloseacetatbutyrat, Butylmethacrylat-2-Dimethylaminoethyl-methacrylat-Methylmethacrylat-Copolymer, Ethylacrylat-Methylmethacrylat-Copolymer, Methacrylsäure-Methylmethacrylat-Copolymer, Methacrylsäure-Ethylacrylat-Copolymer, Methacrylsäure-Methylmethacrylat-Copolymer, Methylacrylat-Methylmethacrylat-Methacrylsäure-Copolymer, Ethylacrylat-Methylmethacrylat-Trimethylamminoethylmethacrylatchlorid-Copolymer und/oder Mischungen davon umfassenden Gruppe versehen sind.

**24.** Pharmazeutische Zubereitung nach Anspruch 23, **dadurch gekennzeichnet, daß** die speichelresistente Außenschicht Aromastoffe, Geschmacksstoffe und/oder Süßungsmittel enthält.

**25.** Pharmazeutische Zubereitung nach mindestens einem der vorhergehenden Ansprüche, **dadurch gekennzeichnet,**

**daß** die mit dem Wirkstoff, den Diffusionsschichten, der magensaftresistenten Überzugsschicht und gegebenenfalls der speichelresistenten Außenschicht versehenen Wirkstoffträger unter Verwendung üblicher Hilfsstoffe zu Tabletten verpreßt oder in Kapseln aus Gelatine, Cellulose, Stärke oder Stärkederivaten abgefüllt oder in Flüssigkeiten oder halbfeste oder feste Zubereitungen zur Herstellung einer Suspension oder eines Suspensionsgels eingebracht sind.

26. Verfahren zur Herstellung von der pharmazeutischen Zubereitung nach den Ansprüchen 1 bis 25, **dadurch gekennzeichnet, daß** man unter Anwendung an sich bekannter Verfahrensweisen die Wirkstoffträger mit einer Lösung oder Suspension, die den Wirkstoff, das Bindemittel, das Trennmittel und gegebenenfalls Puffersubstanzen enthält, beschichtet, dann nacheinander die mindestens zwei Diffusionsschichten, deren Durchlässigkeit für den diffundierenden Wirkstoff von innen nach außen abnimmt, aufbringt, die magensaftresistente Überzugsschicht aufträgt und gegebenenfalls mit einer speichelresistenten Außenschicht versieht.

**Claims**

1. Pharmaceutical preparation for oral administration with controlled release of active ingredient in the small bowel, on the basis of active ingredient carriers provided with at least one active ingredient which are provided with an inner layer to control the release of active ingredient and with a gastro-resistant coating layer disposed thereon, **characterized in that** the inner layer is formed from at least two diffusion layers whose permeability for the diffusing active ingredient decreases from the inside to the outside, the diffusion layers being formed from a matrix material which is insoluble in small bowel fluid and into which pore formers which control the permeability for the diffusing active ingredient and are soluble and/or swellable and/or water-absorbing in small bowel fluid are incorporated and **in that** the pore former of the diffusion layers is selected from the group consisting of polyvinyl alcohol-polyethylene glycol copolymers, polyvinylpyrrolidone, vinylpyrrolidone-vinyl acetate copolymers, crosslinked polyvinylpyrrolidone, microcrystalline cellulose, hydroxypropylcellulose, sodium carboxymethylcellulose, methylcellulose, hydroxyethylcellulose, methylethylcellulose, methylhydroxypropylcellulose, hydroxypropylmethylcellulose, hydroxyethylmethylpropylcellulose, polyethylene glycol, cellulose powder, sucrose, lactose, mannitol, sorbitol and polysorbate, and **in that** the active ingredient carriers comprise as active ingredient 4-diethylamino-2-butynyl $\alpha$-phenylcyclohexaneglyconate hydrochloride, ethyldimethyl(1-methyl-3,3-diphenylpropyl)ammonium carrageenate, (+)-[R-2-[$\alpha$-2-(diisopropylamino)ethyl]benzyl]-p-cresol tartrate, 8-(cyclopropylmethyl)-6$\beta$,7$\beta$-epoxy-3$\alpha$-hydroxy-1$\alpha$H,5$\alpha$)-(S)-tropate bromide, (1R,3r,5S)-3-[(hydroxyldiphenylacetyl)oxy]spiro[8-azoniabicyclo[3.2.1]octane-8,1'-pyrrolidinium] chloride, 2-diethylaminoethyl (bicyclohexyl)-1-carboxylate hydrochloride, 1-methyl-4-piperidyl diphenylpropoxyacetate hydrochloride, (3R)-1-azabicyclo[2.2.2]oct-3-yl (1S)-1-phenyl-3,4-dihydroisoquinoline-2(1H)-carboxylate succinate, (S)-1-[2-(2,3-dihydro-5-benzofuranyl)ethyl]-$\alpha$,$\alpha$-diphenyl-3-pyrrolidineacetamide hydrobromide, (8R)-3$\alpha$-hydroxy-5-isopropyl-1$\alpha$H,5$\alpha$H-tropanium bromide ($\pm$tropate), 8-butyl-6$\beta$,7$\beta$-epoxy-3$\alpha$-[(S)-3-hydroxy-2-phenylpropanoyloxy] tropanium bromide, (+)-(S)-N-methyl-y-(1-naphthyloxy)-2-thiophenepropylamine, 2-[(1R)-3-(diisopropylamino)-1-phenylpropyl]-4-(hydroxymethyl)phenyl isobutyrate, 4-(diethylamino)-but-2-yn-1-yl (2S)-cyclohexyl(hydroxy)phenylacetate, ethyldimethyl(1-methyl-3,3-diphenylpropyl)ammonium bromide, 2-piperidinoethyl 3-methyl-4-oxo-2-phenyl-4H-chromene-8-carboxylate hydrochloride, benzyl(2-chloroethyl)(1-methyl-2-phenoxyethyl)amine hydrochloride and/or a salt or another salt of these active ingredients.

2. Pharmaceutical preparation according to Claim 1, **characterized in that** the diffusion layers comprise identical or different types of matrix materials.

3. Pharmaceutical preparation according to at least either of Claims 1 and 2, **characterized in that** the diffusion layers comprise one or more pore formers which are identical or of different types.

4. Pharmaceutical preparation according to at least one of the preceding claims, **characterized in that** the permeability of the diffusion layers for the diffusing active ingredient is controlled via the nature, the quantity and/or the particle size and/or the solubility and/or the swellability and/or the water-absorbing capacity of the pore formers.

5. Pharmaceutical preparation according to Claim 4, **characterized in that** the inner layer for controlling the release of active ingredient includes an inner and an outer diffusion layer.

6. Pharmaceutical preparation according to Claim 5, **characterized in that** in the case of identical pore former material the particle size and/or the concentration of the pore formers in the inner diffusion layer is greater than in the outer diffusion layer.

EP 1 880 718 B1

7. Pharmaceutical preparation according to Claim 6, **characterized in that** in the case of pore formers which are soluble in small bowel fluid the ratio of the pore former concentration in the inner diffusion layer to the pore former concentration in the outer diffusion layer is in the range from 20:1 to > 1:1, preferably 10:1 to 1.1:1.

8. Pharmaceutical preparation according to at least one of the preceding claims, **characterized in that** the matrix material of the diffusion layers is selected from the group comprising ethylcellulose, cellulose acetate phthalate, cellulose acetate succinate, shellac, hydroxypropylmethylcellulose acetate succinate, cellulose acetate, cellulose acetate propionate, hydroxypropylmethylcellulose phthalate, polyvinyl acetate, polyvinyl acetate phthalate, cellulose acetate butyrate, butyl methacrylate-(2-dimethylaminoethyl) methacrylate-methyl methacrylate copolymers, ethyl acrylate-methyl methacrylate copolymers, methacrylic acid-methyl methacrylate copolymers, methacrylic acid-ethyl acrylate copolymers, ethyl acrylate-methyl methacrylate-trimethyl-ammoniumethyl methacrylate chloride copolymers, chitosan, silicone elastomer latex suspensions, hydrogenated castor oil, stearic acid, glycerol monostearate, glycerol distearate, glycerol dibehenate, stearyl alcohol, white wax, yellow wax, hydrogenated vegetable oil and microcrystalline wax.

9. Pharmaceutical preparation according to at least one of the preceding claims, **characterized in that** the matrix material of the diffusion layers additionally comprises plasticizers and antitack agents.

10. Pharmaceutical preparation according to Claim 9, **characterized in that** the matrix material of the diffusion layers comprises polyethylene glycol, propylene glycol, triethyl citrate, triacetin, acetyl tributyl citrate, polysorbates, 2-pyrrolidone, dibutyl sebacate, stearic acid, castor oil and/or medium-chain triglycerides as plasticizer, and talc, stearic acid and salts thereof, fatty alcohols, mono-, di- or triglycerides with straight- and/or branched-chain fatty acids, colloidal silicon dioxide, precipitated silicon dioxide, aluminium oxide, kaolin, maize starch, wheat starch, rice starch, potato starch, titanium dioxide, silicone emulsions and/or Veegum (a magnesium aluminium silicate dispersion) as antitack agent.

11. Pharmaceutical preparation according to Claim 10, **characterized in that** the active ingredient is present in an amount of from 1 to 250 mg per dose unit on the active ingredient carriers.

12. Pharmaceutical preparation according to Claim 10 or 13, **characterized in that** the active ingredient is present in the form of a coating on the active ingredient carriers.

13. Pharmaceutical preparation according to at least one of the preceding claims, **characterized in that** the active ingredient carriers are in the form of neutral pellets and/or crystalline substances and/or granulated or extruded carrier substances.

14. Pharmaceutical preparation according to Claim 13, **characterized in that** the active ingredient carriers have a particle size of from 0.1 to 3.0 mm.

15. Pharmaceutical preparation according to Claim 13 or 14, **characterized in that** the active ingredient carriers include as carrier material sugar, starch, microcrystalline cellulose, dicalcium phosphate, sodium chloride, citric acid, tartaric acid, malic acid, sucrose, lactose, sorbitol, mannitol, cellulose, calcium hydrogen phosphate, sodium citrate, tricalcium phosphate, maize starch, wheat starch, potato starch, rice starch and/or mixtures thereof.

16. Pharmaceutical preparation according to at least one of the preceding claims, **characterized in that** the active ingredient carriers provided with the active ingredient additionally have, besides the active ingredient, on their surface a binder and where appropriate antitack agent and/or buffer substances.

17. Pharmaceutical preparation according to Claim 16, **characterized in that** the active ingredient carriers provided with the active ingredient comprise per dose unit from 1 to 250 mg active ingredient, from 10 to 500 parts by weight of carrier material, from 1 to 100 parts by weight of binder, from 1 to 100 parts by weight of antitack agent and from 1 to 100 parts by weight of buffer substance.

18. Pharmaceutical preparation according to Claims 16 and 17, **characterized in that** the binder of the active ingredient carriers provided with active ingredient is selected from the group comprising hydroxypropylmethylcellulose, butyl methacrylate-(2-dimethylaminoethyl) methacrylate-methyl methacrylate copolymers, ethyl acrylate-methyl methacrylate copolymers, methacrylic acid-methyl methacrylate copolymers, methacrylic acid-ethyl acrylate copolymers, methyl acrylate-methyl methacrylate-methacrylic acid copolymers, ethyl acrylate-methyl methacrylate-trimethylam-

17

moniumethyl methacrylate chloride copolymers, ethylcellulose, sodium carboxymethylcellulose, methylcellulose, hydroxyethylcellulose, methylethylcellulose, hydroxyethylmethylpropylcellulose, polyvinylpyrrolidone, polyvinyl acetate, vinylpyrrolidone-vinyl acetate copolymers, polyethylene glycol, gelatin, maize starch, wheat starch, rice starch and potato starch and mixtures thereof.

19. Pharmaceutical preparation according to Claims 16 to 18, **characterized in that** the antitack agent of the active ingredient carriers provided with the active ingredient is selected from the group comprising talc, stearic acid and salts thereof, mono-, di-, triglycerides with straight- and/or branched-chain fatty acids, fatty alcohols, colloidal silicon dioxide, precipitated silicon dioxide, aluminium oxide, hydrogenated castor oil, and macrogol (polyethylene glycol) and mixtures thereof.

20. Pharmaceutical preparation according to Claims 16 to 19, **characterized in that** the buffer substances of the active ingredient carriers provided with the active ingredient are selected from the group comprising sodium hydroxide, citric acid, tartaric acid, phosphoric acid, ascorbic acid, succinic acid, adipic acid, fumaric acid and their pharmaceutically acceptable salts and mixtures thereof.

21. Pharmaceutical preparation according to at least one of the preceding claims, **characterized in that** the gastro-resistant coating layer disposed on the active ingredient carrier provided with the active ingredient and the diffusion layers is formed from the group comprising ethylcellulose, cellulose acetate phthalate, cellulose acetate succinate, shellac, hydroxypropylmethylcellulose acetate succinate, cellulose acetate, cellulose acetate propionate, hydroxypropylmethylcellulose phthalate, polyvinyl acetate phthalate, cellulose acetate butyrate, butyl methacrylate-(2-dimethylaminoethyl methacrylate-methyl methacrylate copolymer, ethyl acrylate-methyl methacrylate copolymer, methacrylic acid-methyl methacrylate copolymer, methacrylic acid-ethyl acrylate copolymer, methacrylic acid-methyl methacrylate copolymer, methyl acrylate-methyl methacrylate-mthacrylic acid copolymer, ethyl acrylate-methyl methacrylate-trimethylaminoethyl methacrylate chloride copolymer and/or mixtures thereof.

22. Pharmaceutical preparation according to at least one of the preceding claims, **characterized in that** the active ingredient carriers provided with the active ingredient, the diffusion layers and where appropriate the gastro-resistant coating are provided with a saliva-resistant outer layer.

23. Pharmaceutical preparation according to claim 22, **characterized in that** the active ingredient carriers provided with the active ingredient, the diffusion layers and where appropriate the gastro-resistant coating are provided with a saliva-resistant outer layer from the group comprising ethylcellulose, cellulose acetate phthalate, cellulose acetate succinate, shellac, hydroxypropylmethylcellulose acetate succinate, cellulose acetate, cellulose acetate propionate, hydroxypropylmethylcellulose phthalate, polyvinyl acetate, polyvinyl acetate phthalate, polyvinyl alcohol-polyethylene glycol copolymer, cellulose acetate butyrate, butyl methacrylate-2-dimethylaminoethyl methacrylate-methyl methacrylate copolymer, ethyl acrylate-methyl methacrylate copolymer, methacrylic acid-methyl methacrylate copolymer, methacrylic acid-ethyl acrylate copolymer, methacrylic acid-methyl methacrylate copolymer, methyl acrylate-methyl methacrylate-methacrylic acid copolymer, ethyl acrylate-methyl methacrylate-trimethylaminoethyl methacrylate chloride copolymer and/or mixtures thereof.

24. Pharmaceutical preparation according to Claim 23, **characterized in that** the saliva-resistant outer layer comprises aromatizing substances, flavourings and/or sweeteners.

25. Pharmaceutical preparation according to at least one of the preceding claims, **characterized in that** the active ingredient carriers which have been provided with the active ingredient, the diffusion layers, the gastro-resistant coating layer, and, where appropriate, the saliva-resistant outer layer, are compressed to tablets using conventional excipients, or packed into capsules made of gelatin, cellulose, starch or starch derivatives, or are in the form of liquids or semisolid or solid preparations for preparing a suspension or a suspension gel.

26. Method for producing the pharmaceutical preparation according to Claims 1 to 25, **characterized in that**, using procedures known per se, the active ingredient carriers are coated with a solution or suspension which comprises the active ingredient, the binder, the antitack agent and, where appropriate, buffer substances, then the at least two diffusion layers whose permeability for the diffusing active ingredient decreases from the inside to the outside are successively applied, the gastro-resistant coating layer is applied and, where appropriate, a saliva-resistant outer layer is provided.

**Revendications**

1. Préparation pharmaceutique conçue pour être administrée par voie orale et pour libérer la substance active de façon maîtrisée dans l'intestin grêle, à base de supports de substance active pourvus d'au moins une substance active, lesquels supports sont munis d'une couche interne servant à maîtriser la libération de la substance active et d'une couche d'enrobage résistante au suc gastrique et disposée pardessus la première, **caractérisée en ce**ci :

   - que la couche interne est constituée d'au moins deux couches à diffusion, dont la perméabilité pour la substance active diffusante diminue dans le sens allant de l'intérieur vers l'extérieur, lesquelles couches à diffusion sont constituées d'un matériau matrice qui ne se dissout pas dans le suc gastrique et dans lequel sont incorporés des agents de formation de pores qui peuvent, dans le suc gastrique, se dissoudre et/ou gonfler et/ou absorber de l'eau et qui permettent de maîtriser la perméabilité pour la substance active diffusante ;
   - que l'agent de formation de pores des couches à diffusion est choisi dans l'ensemble formé par les copolymères de type poly(alcool vinylique)-polyéthylèneglycol, la poly(vinyl-pyrrolidone), les copolymères de vinyl-pyrrolidone et d'acétate de vinyle, une poly(vinyl-pyrrolidone) réticulée, une cellulose microcristalline, l'hydroxypropyl-cellulose, la carboxyméthyl-cellulose sodique, la méthyl-cellulose, l'hydroxyéthyl-cellulose, la méthyl-éthyl-cellulose, la méthyl-hydroxypropyl-cellulose, l'hydroxypropyl-méthyl-cellulose, l'hydroxyéthyl-méthyl-propyl-cellulose, le polyéthylèneglycol, la cellulose en poudre, le saccharose, le lactose, le mannitol, le sorbitol, et un polysorbate ;
   - et que les supports de substance active comportent, en tant que substance active, du chlorhydrate de 4-diéthylamino-2-butynyl-$\alpha$-phényl-cyclohexane-gluconate, du carraghénate d'éthyl-diméthyl-(1-méthyl-3,3-diphényl-propyl)-ammonium, du (+)-tartrate de (R)-2-{$\alpha$-[2-(di-isopropylamino)-éthyl]-benzyl}-para-crésol, du bromure de 8-(cyclo-propyl-méthyl)-6$\beta$,7$\beta$-époxy-3$\alpha$-hydroxy-1$\beta$H,5$\alpha$-(S)-tropate, du chlorure de (1R,3R,5S)-3-[(hydroxy-diphényl-acétyl)-oxy]-spiro[8-azonia-bicyclo[3.2.1]octane-8,1'-pyrrolidinium], du chlorhydrate de bicyclo-hexyle-1-carboxylate de 2-(diéthyl-amino)-éthyle, du chlorhydrate de diphényl-propoxy-acétate de 1-méthyl-4-pipéridyle, du succinate de (1S)-1-phényl-3,4-dihydroisoquinoléine-2(1H)-carboxylate de (3R)-1-aza-bicyclo[2.2.2]oct-3-yle, du bromhydrate de (S)-1-[2-(2,3-dihydro-benzofuran-5-yl)-éthyl]-$\alpha$,$\alpha$-diphényl-pyrrolidine-3-acétamide, du bromure de (8R)-3$\alpha$-hydroxy-8-isopropyl-1$\alpha$H,5$\alpha$H-tropanium (($\pm$)-tropate), du bromure de 8-butyl-6$\beta$,7$\beta$-époxy-3$\beta$-((S)-3-hydroxy-2-phényl-propanoyl-oxy)-tropanium, de la (+)-(S)-N-méthyl-(napht-1-yl-oxy)-2-thio-phénpropylamine, de l'isobutyrate de 2-[(1R)-3-(diisopropyl-amino)-1-phényl-propyl]-4-(hydroxyméthyl)-phényle, du (2S)-cyclo-hexyl-phényl-hydroxy-acétate de 4-(diéthylamino)-but-2-yn-1-yle, du bromure d'éthyl-diméthyl-(1-méthyl-3,3-diphényl-propyl)-ammonium, du chlorhydrate de 3-méthyl-4-oxo-2-phényl-4H-chromène-8-carboxy-late de 2-pipéridino-éthyle, ou du chlorhydrate de benzyl-(2-chloro-éthyl)-(1-méthyl-2-phénoxy-éthyl)-amine, et/ou un sel ou un autre sel de ces substances actives.

2. Préparation pharmaceutique conforme à la revendication 1, **caractérisée en ce que** les couches à diffusion comprennent des matériaux matrices qui sont identiques ou différents.

3. Préparation pharmaceutique conforme à l'une au moins des revendications 1 et 2, **caractérisée en ce que** les couches à diffusion contiennent un agent ou plusieurs agents, identiques ou différents, de formation de pores.

4. Préparation pharmaceutique conforme à l'une au moins des revendications précédentes, **caractérisée en ce que** la perméabilité des couches à diffusion pour la substance active diffusante est commandée par la nature, la quantité et/ou la taille des particules et/ou la solubilité et/ou la capacité de gonflement et/ou la capacité d'absorption d'eau des agents de formation de pores.

5. Préparation pharmaceutique conforme à la revendication 4, **caractérisée en ce que** la couche interne servant à maîtriser la libération de la substance active comprend deux couches à diffusion, l'une interne et l'autre externe.

6. Préparation pharmaceutique conforme à la revendication 5, **caractérisée en ce que**, dans le cas où les agents de formation de pores sont le même, la taille de particules et/ou la concentration de l'agent de formation de pores dans la couche à diffusion interne est/sont plus grande(s) que dans la couche à diffusion externe.

7. Préparation pharmaceutique conforme à la revendication 6, **caractérisée en ce que**, dans le cas où les agents de formation de pores sont solubles dans du suc intestinal, le rapport de la concentration d'agent de formation de pores dans la couche à diffusion interne à la concentration d'agent de formation de pores dans la couche à diffusion externe vaut de 20/1 à plus de 1/1, et de préférence de 10/1 à 1,1/1.

**8.** Préparation pharmaceutique conforme à l'une au moins des revendications précédentes, **caractérisée en ce que** le matériau matrice des couches à diffusion est choisi dans l'ensemble constitué par les suivants : éthyl-cellulose, acétate-phtalate de cellulose, acétate-succinate de cellulose, gomme-laque, acétate-succinate d'hydroxypropyl-méthyl-cellulose, acétate de cellulose, acétate-propionate de cellulose, phtalate d'hydroxypropyl-méthyl-cellulose, poly(acétate de vinyle), poly(acétate-phtalate de vinyle), acétate-butyrate de cellulose, copolymère de méthacrylate de butyle, de méthacrylate de 2-(diméthylamino)-éthyle et de méthacrylate de méthyle, copolymère d'acrylate d'éthyle et de méthacrylate de méthyle, copolymère d'acide méthacrylique et de méthacrylate de méthyle, copolymère d'acrylate d'éthyle et d'acide méthacrylique, copolymère d'acrylate d'éthyle, de méthacrylate de méthyle et de chlorure de triméthyl-(méthacryloyloxy-éthyl)-ammonium, chitosane, suspensions latex d'élastomères silicones, huile de ricin hydrogénée, acide stéarique, monostéarate de glycérol, distéarate de glycérol, dibéhénate de glycérol, alcool stéarylique, cire blanche, cire jaune, huile végétale hydrogénée et cire microcristalline.

**9.** Préparation pharmaceutique conforme à l'une au moins des revendications précédentes, **caractérisée en ce que** le matériau matrice des couches à diffusion contient en outre un plastifiant et un agent de démoulage.

**10.** Préparation pharmaceutique conforme à la revendication 9, **caractérisée en ce que** le matériau matrice des couches à diffusion contient, en tant que plastifiant, un polyéthylèneglycol, du propylène-glycol, du citrate de triéthyle, de la triacétine, de l'acétyl-citrate de tributyle, un polysorbate, de la 2-pyrrolidone, de l'ester dibutylique d'acide sébacique, de l'acide stéarique, de l'huile de ricin et/ou des triglycérides à chaînes moyennes, et en tant qu'agent de démoulage, du talc, de l'acide stéarique ou de l'un de ses sels, un alcool gras, un monoglycéride, diglycéride ou triglycéride d'acide(s) gras à chaîne(s) droite(s) et/ou ramifiée(s), de la silice très dispersée, de la silice précipitée, de l'alumine, du kaolin, de l'amidon de maïs, de l'amidon de blé, de l'amidon de riz, de l'amidon de pomme de terre, du dioxyde de titane, une émulsion de silicone et/ou du produit Veegum (dispersion d'alumino-silicate de magnésium).

**11.** Préparation pharmaceutique conforme à la revendication 10, **caractérisée en ce que** la substance active se trouve, sur ou dans les supports de substance active, en une quantité de 1 à 250 mg par dose unitaire.

**12.** Préparation pharmaceutique conforme à la revendication 10 ou 11, **caractérisée en ce que** la substance active se présente sous la forme d'un revêtement déposé sur les supports de substance active.

**13.** Préparation pharmaceutique conforme à l'une au moins des revendications précédentes, **caractérisée en ce que** les supports de substance active se présentent sous forme de pastilles neutres et/ou de substances cristallines et/ou de substances de support extrudées ou granulées.

**14.** Préparation pharmaceutique conforme à la revendication 13, **caractérisée en ce que** les supports de substance active présentent une taille de particules de 0,1 à 3,0 mm.

**15.** Préparation pharmaceutique conforme à la revendication 13 ou 14, **caractérisée en ce que** les supports de substance active comprennent, en tant que matériau support, du sucre, de l'amidon, de la cellulose microcristalline, du phosphate dicalcique, du chlorure de sodium, de l'acide citrique, de l'acide tartrique, de l'acide malique, du saccharose, du lactose, du sorbitol, du mannitol, de la cellulose, de l'hydrogénophosphate de calcium, du citrate de sodium, du phosphate tricalcique, de l'amidon de maïs, de l'amidon de blé, de l'amidon de pomme de terre, de l'amidon de riz, et/ou de l'un de leurs mélanges.

**16.** Préparation pharmaceutique conforme à l'une au moins des revendications précédentes, **caractérisée en ce que** les supports de substance active pourvus de la substance active portent à leur surface, en plus de la substance active, un liant ainsi que, le cas échéant, un agent de démoulage et/ou une substance tampon.

**17.** Préparation pharmaceutique conforme à la revendication 16, **caractérisée en ce que en ce que** les supports de substance active pourvus de la substance active contiennent, par dose unitaire, 1 à 250 mg de substance active, 10 à 500 parties en poids de matériau support, 1 à 100 parties en poids de liant, 1 à 100 parties en poids d'agent de démoulage, et 1 à 100 parties en poids de substance tampon.

**18.** Préparation pharmaceutique conforme aux revendications 16 et 17, **caractérisée en ce que** le liant des supports de substance active pourvus de la substance active est choisi dans l'ensemble formé par les suivants : hydroxypropyl-méthyl-cellulose, copolymère de méthacrylate de butyle, de méthacrylate de 2-(diméthylamino)-éthyle et de méthacrylate de méthyle, copolymère d'acrylate d'éthyle et de méthacrylate de méthyle, copolymère d'acide méthacrylique et de méthacrylate de méthyle, copolymère d'acide méthacrylique et d'acrylate d'éthyle, copolymère d'acrylate de méthyle, de méthacrylate de méthyle et d'acide méthacrylique, copolymère d'acrylate d'éthyle, de méthacrylate de méthyle et de chlorure de triméthyl-(méthacryloyloxy-éthyl)-ammonium, éthyl-cellulose, carboxyméthyl-cellulose sodique, méthyl-cellulose, hydroxyéthyl-cellulose, méthyl-éthyl-cellulose, hydroxyéthyl-méthyl-propyl-cellulose, poly(vinyl-pyrrolidone), poly(acétate de vinyle), copolymère de vinyl-pyrrolidone et d'acétate de vinyle, polyéthylène-glycol, gélatine, amidon de maïs, amidon de blé, amidon de riz, amidon de pomme de terre, et leurs mélanges.

19. Préparation pharmaceutique conforme aux revendications 16 à 18, **caractérisée en ce que** l'agent de démoulage des supports de substance active pourvus de la substance active est choisi dans l'ensemble formé par les suivants : talc, acide stéarique et ses sels, monoglycé-rides, diglycérides et triglycérides d'acide(s) gras à chaîne(s) droite(s) et/ou ramifiée(s), alcools gras, silice très dispersée, silice précipitée, alumine, huile de ricin hydrogénée, produit Macrogol (polyéthylèneglycol), et leurs mélanges.

20. Préparation pharmaceutique conforme aux revendications 16 à 19, **caractérisée en ce que** la substance tampon des supports de substance active pourvus de la substance active est choisie dans l'ensemble formé par les suivants : hydroxyde de sodium, acide citrique, acide tartrique, acide phosphorique, acide ascorbique, acide succinique, acide adipique, acide fumarique, ainsi que leurs sels pharmacologique-ment admissibles, et leurs mélanges.

21. Préparation pharmaceutique conforme à l'une au moins des revendications précédentes, **caractérisée en ce que** la couche d'enrobage résistante au suc gastrique et placée sur le support de substance active pourvu de la substance active et des couches à diffusion est faite d'un matériau choisi dans l'ensemble formé par les suivants : éthyl-cellulose, acétate-phtalate de cellulose, acétate-succinate de cellulose, gomme-laque, acétate-succinate d'hydroxypropyl-méthyl-cellulose, acétate de cellulose, acétate-propionate de cellulose, phtalate d'hydroxypropyl-méthyl-cellulose, poly(acétate-phtalate de vinyle), acétate-butyrate de cellulose, copolymère de méthacrylate de butyle, méthacrylate de 2-(diméthylamino)-éthyle et méthacrylate de méthyle, copolymère d'acrylate d'éthyle et de méthacrylate de méthyle, copolymère d'acide méthacrylique et de méthacrylate de méthyle, copolymère d'acide méthacrylique et d'acrylate d'éthyle, copolymère d'acrylate de méthyle, de méthacrylate de méthyle et d'acide méthacrylique, et copolymère d'acrylate d'éthyle, de méthacrylate de méthyle et de chlorure de triméthyl-(méthacryloyloxy-éthyl)-ammonium, ainsi que leurs mélanges

22. Préparation pharmaceutique conforme à l'une au moins des revendications précédentes, **caractérisée en ce que** les supports de substance active pourvus de la substance active, des couches à diffusion et, le cas échéant, de l'enrobage résistant au suc gastrique sont munis d'une couche externe résistante à la salive.

23. Préparation pharmaceutique conforme à la revendication 22, **caractérisée en ce que** les supports de substance active pourvus de la substance active, des couches à diffusion et, le cas échéant, de l'enrobage résistant au suc gastrique sont munis d'une couche externe résistante à la salive qui est faite d'un matériau choisi dans l'ensemble formé par les suivants : éthyl-cellulose, acétate-phtalate de cellulose, acétate-succinate de cellulose, gomme-laque, acétate-succinate d'hydroxypropyl-méthyl-cellulose, acétate de cellulose, acétate-propionate de cellulose, phtalate d'hydroxypropyl-méthyl-cellulose, poly(acétate de vinyle), poly(acétate-phtalate de vinyle), copolymère poly(alcool vinylique)-polyéthylèneglycol, acétate-butyrate de cellulose, copolymère de méthacrylate de butyle, de méthacrylate de 2-(diméthyl-amino)-éthyle et de méthacrylate de méthyle, copolymère d'acrylate d'éthyle et de méthacrylate de méthyle, copolymère d'acide méthacrylique et de méthacrylate de méthyle, copolymère d'acrylate d'éthyle et d'acide méthacrylique, copolymère d'acrylate de méthyle, de méthacrylate de méthyle et d'acide méthacrylique, et copolymère d'acrylate d'éthyle, de méthacrylate de méthyle et de chlorure de triméthyl-(méthacryloyloxy-éthyl)-ammonium, ainsi que leurs mélanges.

24. Préparation pharmaceutique conforme à la revendication 23, **caractérisée en ce que** la couche externe résistante à la salive contient des agents aromatisants, des agents de sapidité et/ou des agents sucrants.

25. Préparation pharmaceutique conforme à l'une au moins des revendications prédcédentes, **caractérisée en ce que** les supports de substance active pourvus de la substance active, des couches à diffusion, de la couche d'enrobage résistante au suc gastrique et, le cas échéant, de la couche externe résistante à la salive sont mis, avec les adjuvants habituels, sous forme de comprimés ou dans des capsules en gélatine, en cellulose, en amidon ou en dérivés d'amidon, ou incorporés dans des liquides ou dans des préparations solides ou semi-solides en vue de la fabrication d'une suspension ou d'un gel de suspension.

26. Procédé de fabrication d'une préparation pharmaceutique conforme aux revendications 1 à 25, **caractérisé en ce**

**que**, en suivant un mode opératoire connu, on revêt les supports de substance active avec une solution ou une suspension contenant la substance active, le liant, l'agent de démoulage et, le cas échéant, la substance tampon, puis on dépose l'une après l'autre les couches à diffusion, au nombre d'au moins deux, dont la perméabilité pour la substance active diffusante diminue dans le sens allant de l'intérieur vers l'extérieur, on dépose la couche d'enrobage résistante au suc gastrique, et le cas échéant, on munit le tout d'une couche externe résistante à la salive.

Freisetzung des Wirkstoffs (1R,3r,5S)-3-[(Hydroxydiphenylacetyl)-oxy]-spiro[8-azoniabicyclo-[3.2.1]octan-8,1'-pyrrolidinium]-chlorid

Figur 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19823940 A1
- EP 0941070 B1
- US 20040142035 A1
- WO 01088058 A
- EP 1086694 A